# EUROPEAN PATENT SPECIFICATION

(11) **EP 4 645 212 B1**
(45) Date of publication and mention of the grant of the patent: **18.11.2026**
(21) Application number: 24173342.7
(22) Date of filing: 30.04.2024
(51) Int. Cl.: G06T 5/70, G06T 5/50

(54) **DENOISING IN X-RAY IMAGING**
ENTRAUSCHUNG IN DER RÖNTGENBILDGEBUNG
DÉBRUITAGE EN IMAGERIE PAR RAYONS X

(43) Date of publication of application: 05.11.2025
(73) Proprietor: Siemens Healthineers AG, 91301 Forchheim (DE)
(72) Inventor: Hariharan, Sai Gokul, 91301 Forchheim (DE)
(74) Representative: Siemens Healthineers Patent Attorneys

(56) References cited:
- US-B2- 10 692 189
- HARIHARAN SAI GOKUL ET AL: "Robust learning-based x-ray image denoising-potential pitfalls, their analysis and solutions", vol. 8, no. 3, 7 April 2022 (2022-04-07), GB, pages 035013, XP093206252, ISSN: 2057-1976, Retrieved from the Internet <URL:https://iopscience.iop.org/article/10.1088/2057-1976/ac3489/pdf> DOI: 10.1088/2057-1976/ac3489
- WU QIANYU ET AL: "Masked Joint Bilateral Filtering via Deep Image Prior for Digital X-Ray Image Denoising", IEEE JOURNAL OF BIOMEDICAL AND HEALTH INFORMATICS, IEEE, PISCATAWAY, NJ, USA, vol. 26, no. 8, 1 June 2022 (2022-06-01), pages 4008 - 4019, XP011917449, ISSN: 2168-2194, [retrieved on 20220602], DOI: 10.1109/JBHI.2022.3179652
- LI JIATU: "MIDC-Net: Medical Image Denoising and Disease Classification Network for Chest X-rays", 2023 3RD INTERNATIONAL CONFERENCE ON ELECTRONIC INFORMATION ENGINEERING AND COMPUTER SCIENCE (EIECS), IEEE, 22 September 2023 (2023-09-22), pages 834 - 838, XP034552083, DOI: 10.1109/EIECS59936.2023.10435558

## Description

The present invention is directed to a computer-implemented method for denoising in X-ray imaging and to a corresponding computer-implemented training method for supervised training of a denoising algorithm for denoising in X-ray imaging. The invention is further directed to a data processing system adapted to carry out such a computer-implemented method or training method and to an X-ray imaging system comprising such data processing system. The invention is further directed to a corresponding computer program product.

Complex X-ray guided medical procedures may expose the patient and, especially over time, the involved clinical staff to a non-negligible amount of radiation dose. In order to reduce the dose and the risk of potentially correlated health consequences, the dose should be optimized. This means that the applied radiation dose should be as low as reasonably achievable ("ALARA"), while the necessary image quality should be maintained. However, lowering the dose during X-ray imaging results in an increase of noise and thus, a reduction in the signal-to-noise-ratio, SNR, and the image quality. Therefore, it becomes necessary to apply post processing algorithms, in particular denoising techniques, in the imaging chain.

Known denoising algorithms deliver very good results at various dose levels. However, at very low detector entrance dose levels and low SNR levels, they cannot compensate for information loss. That is, the approach removes noise and cannot bring back the information that has not been present in the raw data due to low SNR. In addition, known approaches do not take into consideration the noise characteristics associated with different X-ray spectra.

In the publication S. Hariharan et al.: "Learning-based X-ray Image Denoising Utilizing Model-based Image Simulations", in Shen, D., et al.: "Medical Image Computing and Computer Assisted Intervention - MICCAI 2019.", MICCAI 2019, Lecture Notes in Computer Science, vol. 11769, Springer, Cham, the generalized Anscombe transform is applied to medical X-ray imaging. Furthermore, it is described how a trained artificial neural network can be used as a denoising algorithm based on accordingly transformed X-ray images.

The transformation of the X-ray quanta received by an X-ray detector, for example an indirect-detection, flat-panel detector, into a pixel gray value can be described by a succession of stages. Each stage may for example involve a quantum gain or spatial spreading, also denoted as blurring. It can be assumed that this process follows a linear model. Thus, an observed noise-corrupted gray value y at row r and column c of a detector array of the X-ray detector can be represented as $y \left[r, c\right] = β ⋅ \left(x∗{k}_{q}\right) \left[r, c\right] + g + n$ where *x* represents the charges (corrupted by quantum noise) at the photo-detectors, which convolved with the stochastic spreading function *k_{q}*. The variable n represents electronic noise with a standard deviation, *σₙ* sampled at row position r and column position *c*, respectively. The overall scale factor is given by *β.* The mixed noise variance due to quantum noise and electronic noise of a detector pixel's gray value can be expressed as ${σ}_{y}^{2} = α ⋅ \overline{y} + {σ}_{n}^{2} .$

This can be interpreted as a line with slope *α* and y-intercept ${σ}_{n}^{2}$. The parameter *y̅* denotes the mean, in particular noise-free, value of *y* and the parameter *α* depends on imaging parameters of the X-ray imaging system, which affect the X-ray spectrum, and, for example, a gain factor of the X-ray detector.

In particular, *α* it is affected for example by the X-ray spectrum, the imaged object, a possible spectral X-ray filter, a possible anti-scatter grid, the imaging geometry and, for example, an operating mode of the X-ray imaging system. This makes it difficult to predict the parameters of the noise model just based on the imaging parameters. The parameters *α* and ${σ}_{n}^{2}$ can, for example, be computed directly from an X-ray image, as described for example in the publication S. Hariharan et al.: "Data-driven estimation of noise variance stabilization parameters for low-dose x-ray images.", Phys Med Biol., 2020, 24, 65(22), 225027.

The parameters *α* and ${σ}_{n}^{2}$ can also be obtained from the system specifications of the X-ray imaging system and calibration measurements. Once known, they can be taken into account to perform a noise variance stabilization based on a variance stabilizing transformation, such as the generalized Anscombe transform, GAT, $y ′ = t \left(y, α, {σ}_{n}\right) = \frac{2}{α} \sqrt{α ⋅ y + \frac{3}{8} {α}^{2} + {σ}_{n}^{2}} .$

The GAT makes the noise variance independent of the signal. In fact, the noise variance is stabilized to 1. In addition to the signal dependent noise variance, however, the noise power spectrum also depends on the X-ray spectrum. Unfortunately, the GAT stabilizes only the noise variance and not the noise power spectrum.

In the publication by O. Ronneberger et al.: "U-Net: Convolutional Networks for Biomedical Image Segmentation" (arXiv:1505.04597), the U-Net architecture is described, a widely used CNN architecture for image segmentation, which may, however, also be used for other computer vision tasks.

HARIHARAN SAI GOKUL ET AL: "Robust learning-based x-ray image denoising-potential pitfalls, their analysis and solutions", BIOMEDICAL PHYSICS & ENGINEERING EXPRESS, vol. 8, no. 3, 7 April 2022 (2022-04-07), page 035013, XP093206252, GB, ISSN: 2057-1976, 001: 10.1088/2057-1976/ac3489, discloses a learning-based denoising strategy involving model-based simulations of low-dose x-ray images during the training phase.

US 10 692 189 B2 describes methods for machine learning sparse image representations with deep unfolding and deploying the machine learnt network to denoise medical images.

It is an objective of the present invention, to improve the quality of denoising in X-ray imaging for different X-ray spectra.

This objective is achieved by the subject matter of the independent claims. Further implementations and preferred embodiments are subject matter of the dependent claims.

The invention is based on the idea to apply a denoising algorithm depending on the at least two noise level parameters of an X-ray imaging system for at least two frequency bands, wherein each noise level parameter is associated with one of the at least two frequency bands.

According to an aspect of the invention, a computer-implemented method for denoising in X-ray imaging is provided. Therein, at least two noise level parameters of an X-ray imaging system for at least two frequency bands are received, wherein each noise level parameter of the at least two noise level parameters is associated with exactly one of the at least two frequency bands. A first X-ray image, which is or has been generated by the X-ray imaging system, is received. A first denoised X-ray image is generated by applying a denoising algorithm depending on the at least two noise level parameters to the first X-ray image.

Unless stated otherwise, all steps of the computer-implemented method may be performed by a data processing system, which comprises at least one data processing device. In particular, the at least one data processing device is configured or adapted to perform the steps of the computer-implemented method. For this purpose, the at least one data processing device may for example store a computer program comprising instructions which, when executed by the at least one data processing device, cause the at least one data processing device to execute the computer-implemented method. The expressions "data processing system" and "at least one data processing device" may be used interchangeably, here and in the following. This holds also for respective expressions derived therefrom.

In case the at least one data processing device comprises two or more data processing devices, certain steps carried out by the at least one data processing device may also be understood such that different data processing devices carry out different steps or different parts of a step. In particular, it is not required that each data processing device carries out the steps completely. In other words, carrying out the steps may be distributed amongst the two or more data processing devices.

From each implementation of the computer-implemented method, a respective implementation of a method for denoising in X-ray imaging, which is not purely computer-implemented, is obtained by including respective steps of generating the X-ray image by the X-ray imaging system.

The X-ray image is, in particular, a two-dimensional X-ray protection image, which displays an object or a part of the object, in particular a patient or a body part of the patient, which is for example, located on a patient table of the X-ray imaging system. This object or the part of the object is denoted as image object in the following.

Each of the at least two noise level parameters is associated with one of the at least two frequency bands, which can be understood such that the total number of at least two noise level parameters is equal to the total number of the at least two frequency bands and for each of the at least two frequency bands, a corresponding noise level parameter is received.

In particular, the noise level parameter quantifies the noise content or noise level expected due to quantum noise and electronic noise in the associated frequency band. For example, the noise level parameter may correspond to the noise variance or noise standard deviation in the respective frequency band.

Since the X-ray image is a two dimensional image, the corresponding noise frequency spectrum is also a two-dimensional frequency spectrum. Consequently, a frequency band can for example be understood as a two-dimensional region, in particular connected region, in the noise frequency domain. In particular, a frequency band may be given by an area limited by two concentric circles in the noise frequency domain or, in other words, by a circular ring or annulus in the noise frequency domain.

The denoising algorithm may, in particular comprise a spatial denoising step and/or a temporal denoising step. The denoising algorithm, in particular the spatial denoising step and/or the temporal denoising step, depends parametrically on the least two noise level parameters. Consequently, the denoising algorithm is more effective and consistent for different X-ray spectra involved in generating the respective X-ray image, and is more robust for varying X-ray spectra, for example during the course of fluoroscopy based interventions.

According to several implementations, the denoising algorithm comprises the application of a variance stabilizing transformation, for example a general Anscombe transformation, GAT, followed by the spatial denoising step and/or the temporal denoising step, followed by the application of the inverse of the variance stabilizing transformation.

Consequently, the denoising algorithm, in particular the spatial denoising step and/or the temporal denoising step, achieve further improved results and, in particular, more consistent results for different X-ray spectra involved in generating the respective X-ray image.

Applying the denoising algorithm to the first X-ray image comprises applying a trained machine learning model, MLM, to input data. The input data comprises the first X-ray image or an image depending on the first X-ray image. The input data comprises metadata, which comprises the at least two noise level parameters.

In general terms, a trained MLM may mimic cognitive functions that humans associate with other human minds. In particular, by training based on training data the MLM may be able to adapt to new circumstances and to detect and extrapolate patterns. Another term for a trained MLM is "trained function".

In general, parameters of an MLM can be adapted or updated by means of training. In particular, supervised training, semi-supervised training, unsupervised training, reinforcement learning and/or active learning can be used. Furthermore, representation learning, also denoted as feature learning, can be used. In particular, the parameters of the MLMs can be adapted iteratively by several steps of training. In particular, within the training a certain loss function, also denoted as cost function, can be minimized. In particular, within the training of an artificial neural network, ANN, the backpropagation algorithm can be used.

In particular, an MLM can comprise an ANN, a support vector machine, a decision tree and/or a Bayesian network, and/or the MLM can be based on k-means clustering, Q-learning, genetic algorithms and/or association rules. In particular, an ANN can be or comprise a deep neural network, a convolutional neural network or a convolutional deep neural network. Furthermore, an ANN can be an adversarial network, a deep adversarial network and/or a generative adversarial network.

According to several implementations, the MLM is an ANN, in particular a convolutional neural network, CNN, for example a U-Net.

Such MLMs have proven as particularly suitable image-to-image algorithms in the medical context.

The MLM is, in particular, trained for denoising X-ray images, in particular for spatial denoising. In other words, in such implementations, the denoising algorithm comprises the spatial denoising step and the spatial denoising step is implemented as the trained MLM.

Using the MLM for denoising is particularly beneficial in the framework of the present invention, since MLMs are particularly suitable to take into account additional information, in particular the metadata in the present case, generically without knowing how exactly said additional information affects the denoising processes. This is due to the fact that the MLM has been specifically trained to take into account the metadata in an optimal manner.

The image depending on the first X-ray image on which the MLM is applied may be for example correspond to a variance stabilized X-ray image generated by applying the variance stabilizing transformation to the first X-ray image. In some implementations, alternatively or in addition to the variance stabilizing transformation, the temporal denoising step may be applied to the first X-ray image before applying the MLM. However, in other implementations, the temporal denoising step may also be applied after applying the MLM, in other words may be applied to the output of the MLM. In yet further implementations, the denoising algorithm does not contain the temporal denoising step.

The MLM may be a known MLM for X-ray denoising, which is adapted to be able to process the at least two noise level parameters as metadata. Therein, also the training method for training the MLM may be known in principle and adapted to include also said metadata. For example, the algorithm described in the publication of S. Hariharan et al.: "Learning-based..." may be used as a basis for the MLM.

According to several implementations, the first X-ray image and the metadata are fused to generate fused input data and the MLM is applied to the fused input data.

In other words, the first X-ray image and the metadata are fused at the input level of the MLM and processed jointly by the MLM. Consequently, the metadata is taken into account for generating the first denoised X-ray image in an efficient manner.

The fusion can be carried out in different ways. For example, the metadata may be written into a two-dimensional array and be treated analogously to an additional image channel. Instead of feeding only the first X-ray image, in other words a two-dimensional single-channel image, into the MLM, a two-channel image, whose first channel corresponds to the first X-ray image and whose second channel corresponds to the metadata, is fed to the MLM. However, also alternative fusion methods may be used.

According to several implementations, image features are generated by applying a first part of the MLM to the first X-ray image, the image features and the metadata are fused to generate fused features. The first denoised X-ray image is generated by applying a second part of the MLM to the fused features.

In other words, the first X-ray image and the metadata are fused at an intermediate feature level of the MLM. Consequently, the metadata is taken into account for generating the first denoised X-ray image in an efficient manner. Furthermore, the training of the MLM may be more efficient when the feature extraction is carried out at least partially based on the first X-ray image alone. Furthermore, it may be possible to use an existing feature extraction module as the first part of the MLM without or with less adaptations to take into account also the metadata.

According to several implementations, the first X-ray image corresponds to a first frame of a plurality of consecutive frames. A second X-ray image generated by the X-ray imaging system is received, wherein the second X-ray image corresponds to a second frame of the plurality of consecutive frames, which succeeds the first frame, in particular succeeds the first frame directly or immediately. A difference image corresponding to a difference, for example a pixel-wise difference, between the first X-ray image and the second X-ray image is computed. A frequency decomposition is carried out to generate at least two respective variance maps of the difference image according to the at least two frequency bands. The denoising algorithm is applied to the first X-ray image depending on the at least two variance maps and/or a second denoised X-ray image is generated by applying the denoising algorithm to the second X-ray image depending on the at least two variance maps.

The frequency decomposition may for example be carried out by transforming the difference image into the frequency domain, for example by using a fourier transform or a Laplacian transform or the method of a Laplacian pyramid, et cetera. In the frequency domain, the transformed difference image may be separated into respective frequency maps corresponding to the at least two frequency bands respectively. The frequency maps may then be back transformed into the image domain. The variance maps may then be generated by computing the respective variance of the back transformed frequency maps. Also other approaches for the frequency decomposition are possible.

As a result, the at least two variance maps represent the variance of the difference between the first X-ray image and the second X-ray image in the at least two frequency bands. The denoising algorithm can then specifically take into account the different noise levels in the different frequency bands for the denoising, which leads to an improved quality of the denoising.

According to several implementations, each of a plurality of image regions of the second X-ray image is classified as a static region or as a dynamic region depending on the at least two variance maps using the respective noise level parameter as a classification threshold. The denoising algorithm comprises the temporal denoising step with an adjustable temporal denoising strength and the temporal denoising strength is adjusted depending on a result of the classification for applying the temporal denoising step to the second X-ray image or to an image depending on the second X-ray image.

In particular command the denoising algorithm may also comprise the spatial denoising step, which may for example be carried out prior to the temporal denoising step or afterwards. In particular, the image depending on the second X-ray image on which the temporal denoising step is applied may be generated by means of the spatial denoising step and, in some implementations, the variance stabilizing transformation.

The image regions may be connected groups of pixels, for example pixels within respective rectangular regions of the second X-ray image. However, the second X-ray image may also be partitioned in a different manner into the plurality of image regions.

A static image region may be understood as an image region, whose content does not change or not significantly change when comparing the first X-ray image to the second X-ray image. Analogously, a dynamic image region is an image region, which is not a static image region. Consequently, by adjusting the temporal denoising strength depending on the result of the classification allows to achieve an optimal trade-off between noise reduction and information loss due to the temporal denoising for each image region of the second X-ray image.

The temporal denoising step may for example be carried out by averaging over the corresponding image regions for two or more consecutive frames including the second frame. The averaging may also be a weighted averaging. The temporal denoising strength may for example be adjusted by adjusting the number of frames taken into account for the averaging and/or by adjusting the relative weights of the different frames.

Using the respective noise level parameter as a classification threshold may for example be understood such that the variance of the image region under consideration in the respective variance map is compared with the respective noise level parameter. If said variance is larger than twice the respective noise level parameter or larger than the noise level parameter plus a predefined tolerance, then the corresponding image region may for example be classified as dynamic and otherwise as static.

In some implementations, the temporal denoising step may analogously be applied to the first X-ray image.

According to several implementations, if a number of image regions classified as static regions is larger than a predefined upper threshold value, the temporal denoising strength is adjusted to a predefined upper temporal denoising strength and the temporal denoising step is applied to all of the plurality of image regions according to the upper temporal denoising strength.

In other words, in case the number of image regions classified as static regions is larger than the upper threshold value, then the whole second X-ray image may be considered as a static image and the same temporal denoising strength may uniformly be used for the whole second X-ray image. In this way, computational effort and time may be reduced.

In some implementations, the temporal denoising step may analogously be applied to the first X-ray image.

According to several implementations, if the number of image regions classified as static regions is less than a predefined lower threshold value, which is less than the upper threshold value, the temporal denoising strength is adjusted to a predefined lower temporal denoising strength, which is smaller than the predefined upper temporal denoising strength. The temporal denoising step is applied to all of the plurality of image regions according to the lower temporal denoising strength.

In other words, in case the number of image regions classified as static regions is less than the lower threshold value, then the whole second X-ray image may be considered as a dynamic image and the same temporal denoising strength may uniformly be used for the whole second X-ray image. In this way, computational effort and time may be reduced.

It is noted that the lower threshold value may also be zero in some implementations. Also the lower temporal denoising strength may be zero in some implementations.

In some implementations, the temporal denoising step may analogously be applied to the first X-ray image.

According to several implementations, if the number of image regions classified as static regions is less than the upper threshold value and larger than the lower threshold value, wherein the lower threshold value may be zero in some implementations, then the temporal denoising step is applied to all static image regions according to the upper temporal denoising strength and the temporal denoising step is applied to all dynamic image regions according to the lower temporal denoising strength or the temporal denoising step is not applied to any of the dynamic image regions.

In other words, in case the second X-ray image is mixed in the sense that it both contains static image regions and dynamic image regions to some degree, the static image regions and the dynamic image regions are treated differently with different temporal denoising strengths.

Consequently, an optimal trade-off between noise reduction and information loss due to the temporal denoising may be achieved for the static image regions as well as for the dynamic image regions.

In some implementations, the temporal denoising step may analogously be applied to the first X-ray image.

According to several implementations, the denoising algorithm comprises the spatial denoising step with an adjustable spatial denoising strength. The spatial denoising step is applied to all dynamic image regions according to a predefined upper spatial denoising strength. The spatial denoising step is applied to all static image regions according to a predefined lower spatial denoising strength, which is less than the upper denoising strength. The upper spatial denoising strength and the lower spatial denoising strength are adjusted such that an overall denoising strength of the temporal denoising step and the spatial denoising step is constant or, in other words, is the same for all image regions of the plurality of image regions.

Consequently, the effectivity of the overall denoising algorithm is equally high for all image regions, while still the information lost due to the temporal denoising step is optimized. The overall denoising strength being constant can be understood such that variations of the overall denoising strength within a predefined tolerance range are allowed.

According to several implementations, a set of imaging parameters of the X-ray imaging system corresponding to the generation of first X-ray image and, in respective implementations, the second X-ray image, is received. The at least two noise level parameters are determined depending on the set of imaging parameters, in particular directly or indirectly.

The set of imaging parameters is given by a respective set of imaging parameters, which have been used or were present or were applied when generating the respective X-ray image by means of the X-ray imaging system. The set of imaging parameters may comprise, in particular, any parameters or conditions, which affect or potentially affect the noise content, in particular, the quantum noise present in the X-ray image, and/or the X-ray spectrum. This may include exposure parameters, geometrical parameters, detector parameters of the X-ray detector, parameters of the imaged object, and so forth.

For example, denoting the variance of the total noise by σ² = σ_{q}² + σₙ², wherein σ_{q}² denotes the variance of the quantum noise and σₙ² denotes the variance of the electronic noise, the noise level function, NLF, may be denoted by σ² = α·yₘ + σₙ², with α = (σ² - σₙ²*)* / yₘ. Therein, yₘ corresponds to the mean signal in the resulting X-ray image. The parameter α depends on the X-ray spectrum and, consequently, on set of imaging parameters.

For example, α may also depend on the sensitivity of the pixels of the detector array. The sensitivity of the pixels may for example taken into consideration via calibration images acquired at different X-ray spectra. Instead of a single α, it is also possible to use a map of α values: α[r, c] is then the value of α at the pixel location [r ,c]. Analogously, a map for the electronic noise can also be used: σₙ²[r,c].

In some implementations, the parameter α may be determined based on the set of imaging parameters and the at least two noise level parameters may be determined depending on the parameter α. In practice, for example a database or lookup table relating the set of imaging parameters and/or the parameter α to the at least two noise level parameters may be provided. This is particularly beneficial to implement a real-time execution of the computer-implemented method.

According to several implementations, the variance-stabilizing transformation is a generalized Anscombe transformation.

The generalized Anscombe transformation is on the one hand a well-known variance-stabilizing transformation, which is also applicable in the context of X-ray images, and, on the other hand, has proven particularly suitable for mixtures of Poisson and Gaussian noise. Consequently, a particularly reliable variance stabilization is achieved. The generalized Anscombe transformation is for example given by $y ′ = \frac{2}{α} \sqrt{α ⋅ y + \frac{3}{8} {α}^{2} + {σ}_{n}^{2}} ,$ wherein y denotes a pixel value of the X-ray image, y' denotes the respective pixel value of the noise-variance-stabilized X-ray image, and ${σ}_{n}^{2}$ denotes the variance of the electronic noise of the X-ray detector, which is predetermined, for example during a calibration phase. In case respective maps are used as mentioned above, this can be understood as $y ′ = \frac{2}{α \left[r, c\right]} \sqrt{α \left[r, c\right] ⋅ y + \frac{3}{8} {α}^{2} \left[r, c\right] + {σ}_{n}^{2} \left[r, c\right]} ,$

Alternatively, other variance-stabilizing transformations of the form $y ′ = f \left(α\right) \sqrt{h ⋅ α + g \left(α, {σ}_{n}^{2}\right)} ,$ may be used, wherein f and g denote functions of *α* and $\left(α, {σ}_{n}^{2}\right)$, respectively, and h is a constant.

According to several implementations, a plurality of variance stabilized reference X-ray images generated by the X-ray imaging system according to respective different sets of imaging parameters is received. An average reference image is generated by averaging the reference X-ray images. For each of the reference X-ray images of the plurality of variance stabilized reference X-ray images, a respective difference reference image corresponding to a difference, in particular a pixel-wise difference, between the respective reference X-ray image and the average reference image is computed. For each of the difference reference images, a frequency decomposition is carried out according to the at least two frequency bands. For each of the at least two frequency bands, the respective noise level parameter of the at least two noise level parameter is computed depending on the noise variance in the respective frequency band of the difference reference images according to the frequency decomposition.

In some implementations, receiving the plurality of a variance stabilized reference X-ray images comprises receiving a plurality of reference X-ray images generated by the X-ray imaging system and applying the variance stabilizing transformation, in particular the GAT, to each of the reference X-ray images.

The set of imaging parameters used for generating the respective X-ray image is different for different reference X-ray images in the sense that at least one imaging parameter of the set of imaging parameters differs. Therefore, each reference X-ray image has a different noise content and, consequently, leads to different noise level parameters in the at least two frequency bands.

According to several implementations, the set of imaging parameters comprises exposure parameters of the X-ray source, for example a peak kilovoltage of the X-ray source used for generating the X-ray image and/or a tube current of the X-ray source used for generating the X-ray image and/or an X-ray pulse duration used for generating the X-ray image.

Exposure parameters like these are known to have a significant impact on the energy spectrum of the X-ray quanta emitted by the X-ray source. Consequently, they also affect the noise content in the X-ray image and, in particular, the variance of the quantum noise and the noise level parameter significantly. Simulating the energy deposition depending on the exposure parameters therefore allows to determine the noise level parameter with increased accuracy and, consequently, makes the variance stabilization more effective.

According to several implementations, the set of imaging parameters comprises filter properties of an X-ray filter of the X-ray imaging system, for example a filter material and/or a filter thickness of the X-ray filter.

The X-ray filter is, in particular, arranged between the X-ray source and the imaged object. The X-ray filter and the X-ray source may for example be part of a source unit of the X-ray imaging system, for example of a C-arm of the X-ray imaging system. The choice of the filter material may for example depend on an anode material of the X-ray source. A common choice for tungsten anodes are copper filters. However, others filter materials may include nickel, aluminum or other metals.

The filter material as well as the filter thickness have a significant impact on the energy spectrum of the X-ray quanta after passing the X-ray filter. Consequently, they also affect the noise content in the X-ray image and, in particular, the variance of the quantum noise and the noise level parameter significantly. Therefore the at least two noise level parameters may be determined with increased accuracy.

According to several implementations, the set of imaging parameters comprises an X-ray dose at the X-ray detector.

The X-ray dose at the X-ray detector significantly affects the characteristics of noise at the detector. At very low dose levels, electronic noise can dominate the quantum noise and hence noise in different frequencies can differ. Therefore the at least two noise level parameters may be determined with increased accuracy in such implementations.

According to several implementations, the set of imaging parameters comprises collimator properties of an X-ray collimator of the X-ray imaging system, for example a collimator opening size of the X-ray collimator.

The X-ray collimator is, in particular, arranged between the X-ray filter and the imaged object. The X-ray collimator may for example be part of the source unit of the X-ray imaging system. The choice of the collimator opening size may for example depend on the part of the object, which shall be imaged.

The collimator opening size has a significant impact for example on the scattering of X-ray quanta at the collimator and, since the scattering is energy dependent, also on the energy spectrum of the X-ray quanta after passing the X-ray filter. Consequently, it also affects the noise content in the X-ray image and, in particular, the variance of the quantum noise and the noise level parameters significantly. Therefore the at least two noise level parameters may be determined with increased accuracy.

According to several implementations, the set of imaging parameters comprises a zoom factor used for generating the X-ray image.

The zoom factor corresponds to a part of a detector array of the X-ray detector, whose data is used for generating the X-ray image, and which may be smaller than the full detector array. While the zoom factor may have a smaller effect on the noise content of the X-ray image than the exposure parameters, the filter properties and the collimator properties, the effect may not be neglectable in some applications. Therefore the at least two noise level parameters may be determined with increased accuracy.

According to several implementations, the set of imaging parameters comprises geometrical parameters of the X-ray imaging system and the imaged object, respectively.

The geometrical parameters may for example comprise a position and/or an orientation of the X-ray source with respect to the imaged object and/or a position and/or orientation of the X-ray detector with respect to the imaged object. For example, in particular in case the X-ray imaging system comprises a C-arm carrying the X-ray source and the X-ray detector, the geometrical parameters may comprise a position and/or an angulation, in particular an angular position and an orbital position, of the C-arm.

The geometrical parameters may for example comprise, in some implementations, a position and/or an orientation of a patient table, on which the imaged object is located, or a part of the patient table.

The geometrical parameters may also comprise an effective thickness of the imaged object, for example in terms of a water equivalent thickness, WET.

The geometrical parameters have a significant impact for example on the scattering and transmission and, for example, absorption of X-ray quanta by the imaged object and, in some cases, further objects in the beam path. Consequently, they also affect the noise content in the X-ray image and, in particular, the variance of the quantum noise and the noise level parameters significantly. Therefore the at least two noise level parameters may be determined with increased accuracy.

According to several implementations, the set of imaging parameters comprises a gain factor of the X-ray detector and/or a status parameter of an anti-scattering grid of the X-ray imaging system. The status parameter of the anti-scattering grid may for example be whether the anti-scattering grid is in the beam path or not.

The gain factor and the presence of the anti-scattering grid in the beam path have a significant impact for example on the noise content in the X-ray image and, in particular, the variance of the quantum noise and the noise level parameters. Therefore the at least two noise level parameters may be determined with increased accuracy.

According to several implementations, the set of imaging parameters comprises a pixel sensitivity of the detector pixels of the X-ray detector.

The sensitivity of the detector pixels may for example be taken into consideration via calibration images acquired at different X-ray spectra.

According to a further aspect of the invention, an X-ray imaging method is provided. Therein, a first X-ray image is generated by an X-ray imaging system, in particular by an X-ray source and an X-ray detector of the X-ray imaging system, for example controlled by a control system of the X-ray imaging system. A computer-implemented method for denoising in X-ray imaging according to the invention is carried out based on the first X-ray image, for example by the control system and/or the data processing system, and the first denoised X-ray image or an image depending on the first denoised X-ray image, for example the back transformed denoised X-ray image, is displayed by a display device, for example a display device of the X-ray imaging system.

According to several implementations, the X-ray imaging method is carried out as a fluoroscopy method, wherein a sequence of consecutive X-ray images including said first X-ray image is generated by the X-ray imaging system and each of the consecutive X-ray images is denoised by using a computer-implemented method for denoising in X-ray imaging according to the invention. Each denoised X-ray image or an image depending on the respective denoised X-ray image, is for example displayed by the display device.

Further implementations of the X-ray imaging method according to the invention follow directly from the various embodiments of the computer-implemented method for denoising in X-ray imaging according to the invention and vice versa. In particular, individual features and corresponding explanations as well as advantages relating to the various implementations of the computer-implemented method for denoising in X-ray imaging according to the invention can be transferred analogously to corresponding implementations of the X-ray imaging method according to the invention.

According to a further aspect of the invention, a computer-implemented training method for supervised training of a denoising algorithm, an MLM, for denoising in X-ray imaging is provided. Therein, at least two noise level parameters of an X-ray imaging system for at least two frequency bands are received, wherein each noise level parameter is associated with exactly one of the at least two frequency bands. A loss function for the supervised training depends on the at least two noise level parameters.

In particular, known methods for supervised training of an MLM, in particular an ANN, for example CNN, may be used, wherein the original loss function is replaced by a corresponding loss function depending on the at least two noise level parameters. In this way, the MLM learns to optimally take into account at least two noise level parameters for denoising an X-ray image. The trained denoising algorithm will therefore be able to achieve a more effective denoising consistently for various X-ray spectra.

For example, the denoising algorithm, for example MLM, trained by means of the computer-implemented training method according to the invention, is an algorithm for spatial denoising and, for example, corresponds to the spatial denoising step mentioned above.

According to several implementations, the loss function comprises a weighted sum of errors according to the at least two frequency bands, wherein respective weighting factors of the weighted sum depend on or are given by the at least two noise level parameters.

In other words, the denoised X-ray image obtained during the training is compared to the corresponding ground truth individually for each of the at least two frequency bands to compute the errors for all of the at least two frequency bands. The loss function contains a corresponding term for each of the at least two frequency bands given by the respective error multiplied with the corresponding weighting factor for the respective noise frequency band. In particular, in known training methods, only a single error for the whole noise frequency spectrum is considered. In some implementations, the same noise function may be used also in the computer-implemented training method, wherein the single error is replaced by the weighted sum of errors.

According to several implementations of the computer-implemented method for denoising in X-ray imaging, in particular such implementations, where the trained MLM is used, the denoising algorithm, in particular the MLM, is or has been trained by using a computer-implemented training method for supervised training according to the invention.

According to a further aspect of the invention, a data processing system, which is adapted to carry out a computer-implemented method for denoising in X-ray imaging according to the invention, is provided.

In the present disclosure, the expressions "data processing system" and "at least one data processing device" may be used interchangeably. A data processing device may in particular be understood as a data processing device, which comprises processing circuitry. The data processing device can therefore in particular process data to perform computing operations. This may also include operations to perform indexed accesses to a data structure, for example a look-up table, LUT, as well as a data processing process implemented in hardware.

In particular, the data processing device may include one or more computers, one or more microcontrollers, and/or one or more integrated circuits, for example, one or more application-specific integrated circuits, ASIC, one or more field-programmable gate arrays, FPGA, and/or one or more systems on a chip, SoC. The data processing device may also include one or more processors, for example one or more microprocessors, one or more central processing units, CPU, one or more graphics processing units, GPU, and/or one or more signal processors, in particular one or more digital signal processors, DSP. The data processing device may also include a physical or a virtual cluster of computers or other of said units.

In various embodiments, the data processing device includes one or more hardware and/or software interfaces and/or one or more memory units.

A memory unit may be implemented as a volatile data memory, for example a dynamic random access memory, DRAM, or a static random access memory, SRAM, or as a non-volatile data memory, for example a read-only memory, ROM, a programmable read-only memory, PROM, an erasable programmable read-only memory, EPROM, an electrically erasable programmable read-only memory, EEPROM, a flash memory or flash EEPROM, a ferroelectric random access memory, FRAM, a magnetoresistive random access memory, MRAM, or a phase-change random access memory, PCRAM.

According to a further aspect of the invention, a further data processing system, which is adapted to carry out a computer-implemented training method according to the invention, is provided.

According to a further aspect of the invention, an X-ray imaging system is provided. The X-ray imaging system comprises an X-ray source, an X-ray detector, and a control system, which is configured to control the X-ray source and the X-ray detector to generate a first X-ray image. The X-ray imaging system further comprises a data processing system, which is adapted to carry out a computer-implemented method for denoising in X-ray imaging according to the invention.

The control system and the data processing system may be separated from each other. In this case, the control system may be understood to be or comprise a further data processing system according to the definition above. Alternatively, the data processing system of the X-ray imaging system comprises the control system. In particular, the control system may correspond to one or more data processing apparatuses of the data processing system.

For example, the X-ray imaging system comprises a display device, wherein the control system is configured to control the display device to display the denoised first X-ray image or an image depending on the denoised first X-ray image.

Further implementations of the X-ray imaging system according to the invention follow directly from the various embodiments of the computer-implemented method, the computer-implemented training method and the X-ray imaging method according to the invention and vice versa. In particular, individual features and corresponding explanations as well as advantages relating to the various implementations of the computer-implemented method and the X-ray imaging method according to the invention can be transferred analogously to corresponding implementations of the X-ray imaging system according to the invention. In particular, the X-ray imaging system according to the invention is designed or programmed to carry out the X-ray imaging method according to the invention. In particular, the X-ray imaging system according to the invention carries out the X-ray imaging method according to the invention.

According to a further aspect of the invention, a first computer program comprising first instructions is provided. When the first instructions are executed by a first data processing system, the instructions cause the first data processing system to carry out a computer-implemented method for denoising in X-ray imaging according to the invention.

The first instructions may be provided as program code, for example. The program code can for example be provided as binary code or assembler and/or as source code of a programming language, for example C, and/or as program script, for example Python.

According to a further aspect of the invention, a second computer program comprising second instructions is provided. When the second instructions are executed by an X-ray imaging system according to the invention, in particular by the data processing system of the X-ray imaging system and/or the control system of the X-ray imaging system, the second instructions cause the X-ray imaging system to carry out an X-ray imaging method according to the invention.

The second instructions may be provided as program code, for example. The program code can for example be provided as binary code or assembler and/or as source code of a programming language, for example C, and/or as program script, for example Python.

According to a further aspect of the invention, a third computer program comprising third instructions is provided. When the first instructions are executed by a third data processing system, the instructions cause the third data processing system to carry out a computer-implemented training method according to the invention.

The third instructions may be provided as program code, for example. The program code can for example be provided as binary code or assembler and/or as source code of a programming language, for example C, and/or as program script, for example Python.

According to a further aspect of the invention, a computer-readable storage medium storing a first computer program and/or a second computer program and/or a third computer program according to the invention is provided.

The first computer program, the second computer program, the third computer program and the computer-readable storage medium are respective computer program products comprising the first instructions and/or the second instructions and/or the third instructions.

Above and in the following, the solution according to the invention is described with respect to methods and systems for denoising as well as with respect to methods and systems for providing a trained denoising algorithm. Features, advantages, or alternative embodiments herein can be assigned to the other claimed objects and vice versa. In other words, claims and embodiments for providing a trained denoising algorithm can be improved with features described or claimed in the context denoising in X-ray imaging. In particular, datasets used in the methods and systems can have the same properties and features as the corresponding datasets used in the methods and systems for providing a trained denoising algorithm, and the trained denoising algorithms provided by the respective methods and systems can be used in the methods and systems denoising in X-ray imaging.

Further features and feature combinations of the invention are obtained from the figures and their description as well as the claims. In particular, further implementations of the invention may not necessarily contain all features of one of the claims. Further implementations of the invention may comprise features or combinations of features, which are not recited in the claims.

In the following, the invention will be explained in detail with reference to specific exemplary implementations and respective schematic drawings. In the drawings, identical or functionally identical elements may be denoted by the same reference signs. The description of identical or functionally identical elements is not necessarily repeated with respect to different figures.

In the figures,
- FIG 1: shows schematically an exemplary implementation of an X-ray imaging system according to the invention;
- FIG 2: shows a schematic flow diagram of an exemplary implementation of a computer-implemented method for denoising in X-ray imaging according to the invention;
- FIG 3: shows a schematic flow diagram of a further exemplary implementation of a computer-implemented method for denoising in X-ray imaging according to the invention;

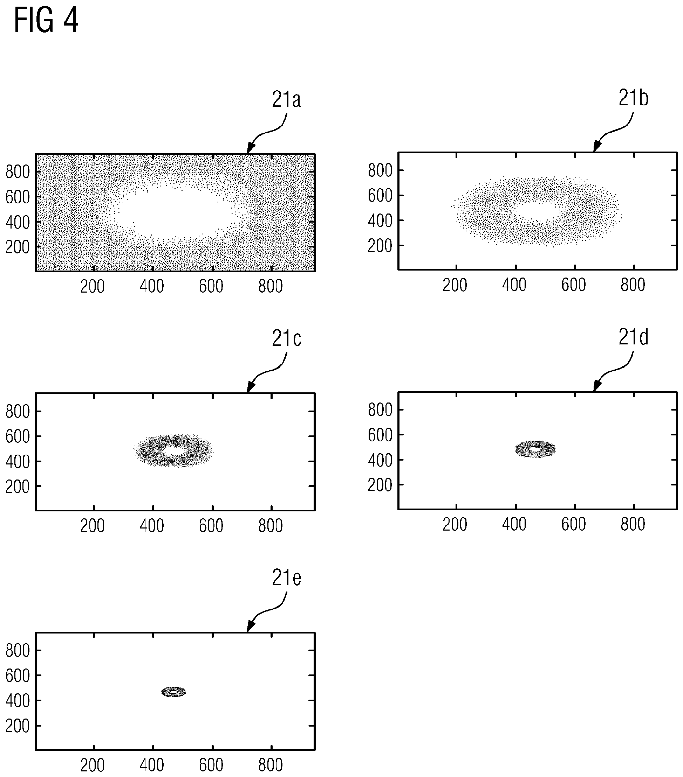

- FIG 4: shows examples of noise in the frequency domain for various bands;
- FIG 5: shows schematically an artificial neural network;
- FIG 6: shows schematically a convolutional neural network; and
- FIG 7: shows schematically a further convolutional neural network.

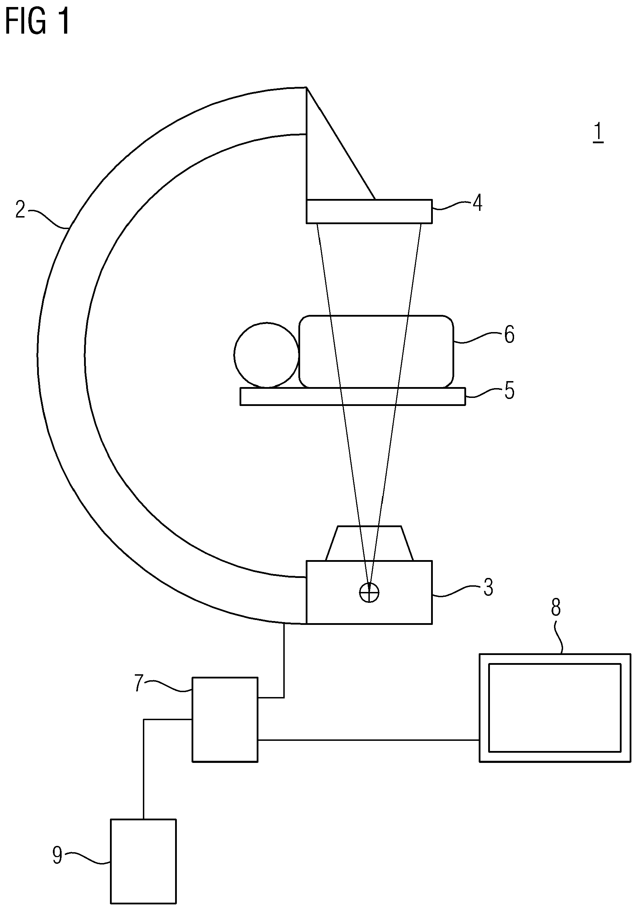

FIG 1 shows schematically an exemplary implementation of an X-ray imaging system 1 according to the invention. The X-ray imaging system 1 comprises a source unit 3 with an X-ray source, a detector unit 4 with an X-ray detector, and a control system 7, which is configured to control the X-ray source and the X-ray detector to generate X-ray images depicting an object 6, for example a patient.

The X-ray imaging system 1 may for example comprise a patient table 5, on which the object 6 is arranged. The X-ray imaging system 1 comprises a data processing system 9 according to the invention, which is adapted to carry out a computer-implemented method for denoising in X-ray imaging according to the invention. In the following, several functions and method steps are described to be carried out by the control system 7, while other functions and method steps are described to be carried out by the data processing system 9. It is noted that the functions and method steps may also be distributed in different ways in alternative implementations.

For example, the control system 7 may adjust various imaging parameters of the X-ray imaging system 1 including, for example, exposure parameters like a peak kilovoltage of the X-ray source, a tube current of the X-ray source, and/or an X-ray pulse duration. For example, the control system 7 may adjust further imaging parameters like a filter material and/or filter thickness of an X-ray filter, for example a copper filter, by placing the appropriate X-ray filter into the beam path or by removing it from the beam path, respectively. For example, the control system 7 may adjust further imaging parameters like a collimator opening size of an X-ray collimator. For example, the control system 7 may bring the X-ray collimator into the beam path or by remove it from the beam path, respectively. For example, the control system 7 may adjust further imaging parameters like a gain factor of the X-ray detector. For example, the control system 7 may bring an anti-scattering grid into the beam path or by remove it from the beam path, respectively.

In some implementations, the X-ray imaging system 1 comprises a display device 8, wherein the control system 7 is configured to control the display device 8 to display X-ray images or processed X-ray images. In case a fluoroscopy-based medical intervention is carried out, the X-ray images are for example generated as a sequence of consecutive frames to allow an operator to monitor or supervise the medical intervention. The X-ray imaging system 1 is, in particular, designed and adapted to carry out an X-ray imaging method according to the invention, wherein an X-ray image is generated by the X-ray imaging system 1 using the X-ray source and the X-ray detector, and a computer-implemented method for denoising in X-ray imaging according to the invention is carried out, in particular by the data processing system 9. The denoised X-ray image is then for example displayed on the display device 8.

In some implementations, the X-ray imaging system 1 is designed as a fluoroscopy system, in particular a C-arm fluoroscopy system, wherein the source unit 3 and the detector unit 4 are attached opposite to each other on a C-arm 2, which can be rotated around different axes. The corresponding motions are denoted as angular and orbital motion respectively. In some implementations, apart from the rotational motion of the C-arm 2, the patient table 5 and the C-arm 2 may be positioned by relative to each other by respective translatory motions of the C-arm 2 and/or the patient table 5. Consequently, the position and/or orientation of the X-ray source with respect to the object 6 and the position and/or orientation of the X-ray detector with respect to the object 6 may be adjusted exactly to the desired imaging perspective.

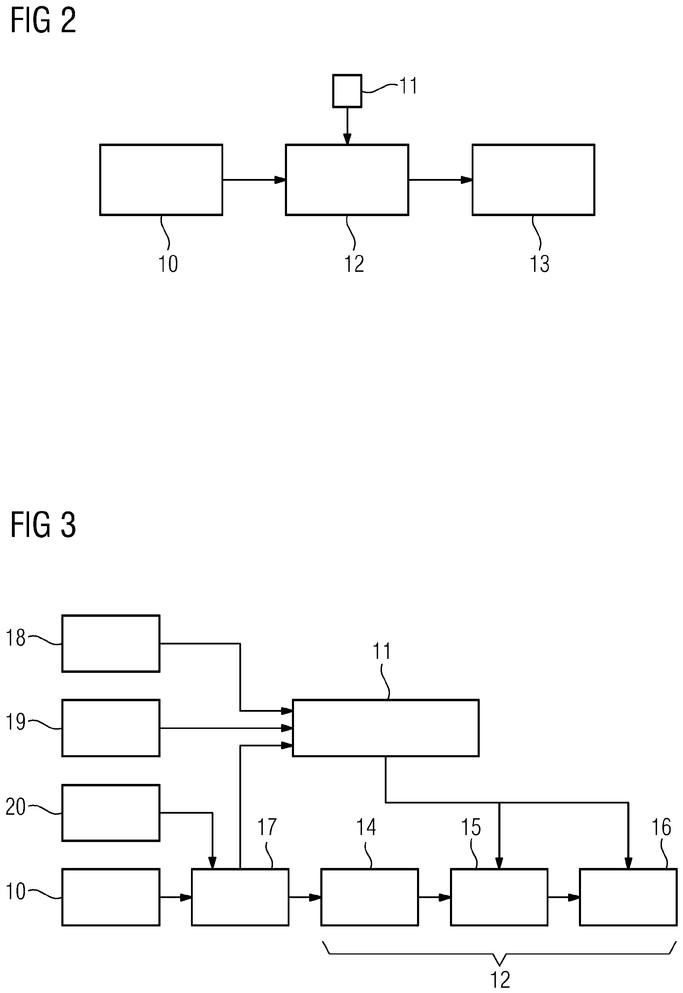

FIG 2 shows a schematic flow diagram of an exemplary implementation of a computer-implemented method for denoising in X-ray imaging according to the invention. Therein, at least two noise level parameters 11 of the X-ray imaging system 1 for at least two frequency bands are received, wherein each noise level parameter 11 is associated with one of the at least two frequency bands. A first X-ray image 10 generated by the X-ray imaging system 1 is received. A first denoised X-ray image 13 is generated by applying a denoising algorithm 12 depending on the at least two noise level parameters 11 to the first X-ray image 10.

FIG 3 shows a schematic flow diagram of a further exemplary implementation of a computer-implemented method for denoising in X-ray imaging according to the invention, which is based on the implementation of FIG 2.

Therein, the NLF, in particular the NLF parameters 17 α and σₑ² of the NLF given by σ² = α·yₘ + σₑ² with α = (σ² - σₑ²) / yₘ and yₘ corresponding to the mean signal are determined. A variance-stabilized X-ray image 14 is generated for each X-ray image 10 of a plurality of consecutive X-ray images generated by the X-ray imaging system 1, in particular by applying a GAT $y ′ = \frac{2}{α} \sqrt{α ⋅ y + \frac{3}{8} {α}^{2} + {σ}_{n}^{2}}$ to each of the X-ray images 10.

Furthermore, imaging parameters 18, 19, 20 of the imaging system 1 are received including, for example exposure parameters 18, geometry parameters 19, and/or detector parameters 20. The at least two noise level parameters 11 may for example be determined depending on the imaging parameters 18, 19, 20 and/or the NLF parameters 17. A spatial denoising step 15 is applied to each of the variance- stabilized X-ray image 14 followed by a temporal denoising step 16. The spatial denoising step 15 may for example be implemented by a trained MLM. The spatial denoising step 15 and/or the temporal denoising step 16 are applied depending on the and the NLF parameters 17. Applying the GAT may be considered as a part of the denoising algorithm 12. This is, however, not necessary. Furthermore, an inverse GAT may be applied to the final output of the spatial denoising step 15 and the temporal denoising step 16.

For example, the noise level parameters 11 may correspond to the noise variance in the respective frequency band. As an example, the variance of noise in five different frequency bands for an X-ray image of a PMMA phantom with a water equivalent thickness, WET, of 30 cm acquired using 81 kVp and a 0.6 mm Cu prefiltration is shown in respective frequency domain images 21a, 21b, 21c, 21d, 21e.

In several implementations of the present invention, a real-time capable hybrid spatio-temporal denoising approach that takes into consideration the noise characteristics associated with a given X-ray spectra in terms of the at least two noise level parameters 11 is provided.

In some implementations, a combination of learning-based classical denoising with temporal averaging based on the at least two noise level parameters 11 is provided.

The input X-ray spectrum, dose, and object thickness do in general change the noise in the different frequency bands. Existing strategies do not take into consideration such variations in the noise. This can result in a sub-optimal noise reduction. Therefore, in the present disclosure, different embodiments for denoising in different frequency bands are proposed, including but not limited to the following embodiments.

In some embodiments, the noise level function parameter α along with the effective object thickness, for example in terms of the WET, exposure parameters and system geometry parameters define the noise level associated with different frequency bands. A high value for α may for example be associated with a hard beam. For example, a thick object absorbs low energy photons and the remaining high energy photons contribute to image formation. Furthermore, it is found that higher energy photons result in a higher α. In addition, α may also give details on the contribution from scattering.

This changes the amount of noise in different frequency bands. Therefore, in case the spatial denoising step 15 is implemented as a trained MLM, the at least two noise level parameters 11 may for example be used during the training and/or during the application of the MLM.

For example, during a supervised training of the MLM, the error in the different frequency bands may weighted based on the at least two noise level parameters 11. These may also be stored as a look-up table and looked up when required. The at least two noise level parameters 11 are passed to the MLM as auxiliary metadata during training and inference.

In some embodiments, the noise level associated with the different frequency bands are used for identifying static and dynamic, that is moving, image regions in the acquired X-ray images 10. After applying the GAT, the variance of the difference between corresponding image regions of a pair of subsequent X-ray images 10 is analyzed in different frequency bands. In case the variance is close to twice the noise variance in the frequency band, the image region may be considered to be static.

For example, respective maps with the variance of differences in the frequency bands are first constructed. These maps are then analyzed to classify the image regions as static and dynamic regions. Strong temporal denoising may be applied for pixels associated with static regions. The maps may be spatially processed to ensure smoothness.

In some embodiments, if most of the regions are identified to be static, which may be defined by a respective threshold setting, strong temporal denoising and weak spatial denoising is applied to the whole image. This is motivated by the reason that strong temporal denoising or even averaging results in an effective dose increase. For example, averaging two X-ray images is almost equivalent to doubling the dose, at least in certain situations.

In some embodiments, if there are static as well as dynamic regions to a relevant extent, which is defined by said threshold setting, strong temporal denoising may be applied in static regions and weaker temporal denoising or no temporal denoising at all may be applied in dynamic regions. Depending on the number of frames used for performing the temporal denoising, the amount of spatial denoising may be adapted to have the same amount of noise reduction across the image in total.

In some embodiments, in the case of spatial denoising based on the trained MLM, the amount of denoising may be controlled within the MLM. Alternatively, the MLM may be designed to remove noise to the maximum extent. Subsequently, a specified amount of the difference between the denoised and the input may be retained. The amount of difference introduced may vary spatially depending on the result of the analysis of said maps.

While known solutions do not account for varying noise levels in different frequency bands due to the X-ray spectrum at the X-ray detector. Several embodiments of the present invention use the signal-dependent noise variance along with details of the X-ray image acquisition, for example exposure parameters, geometry parameters and so forth, to quantify the noise in difference frequency bands.

In some embodiments, this information is used during the development as well as the application of a machine learning-based denoising approach.

In some embodiments the identified noise levels are, alternatively or additionally, used for efficiently guiding the spatio-temporal denoising approach by adaptively making said decisions on when and how to apply spatial denoising, temporal denoising or their combination.

The MLM may for example be an artificial neural network, ANN.

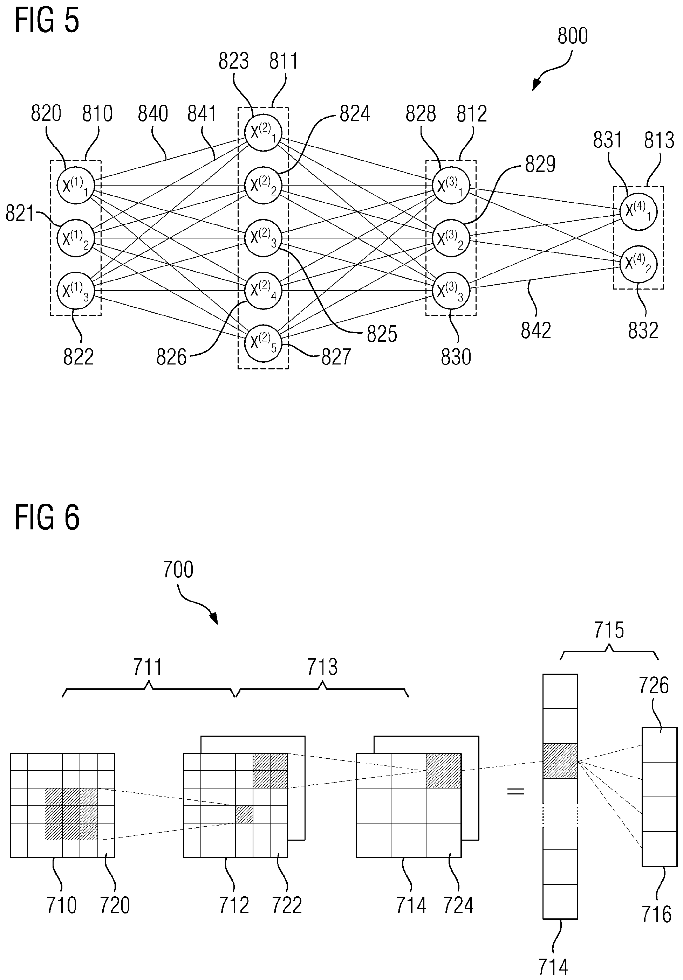

FIG 5 displays an embodiment of an ANN 800, which is for example designed as an MLP. The ANN 800 comprises nodes 820, ..., 832 and edges 840, ..., 842, wherein each edge 840, ..., 842 is a directed connection from a first node 820, ..., 832 to a second node 820, ..., 832. In general, the first node 820, ..., 832 and the second node 820, ..., 832 are different nodes 820, ..., 832. It is, however, also possible that the first node 820, ..., 832 and the second node 820, ..., 832 are identical. For example, in FIG 5, the edge 840 is a directed connection from the node 820 to the node 823, and the edge 842 is a directed connection from the node 830 to the node 832. An edge 840, ..., 842 from a first node 820, ..., 832 to a second node 820, ..., 832 is also denoted as ingoing edge for the second node 820, ..., 832 and as outgoing edge for the first node 820, ..., 832.

In this example, the nodes 820, ..., 832 of the artificial neural network 800 can be arranged in layers 810, ..., 813, wherein the layers can comprise an intrinsic order introduced by the edges 840, ..., 842 between the nodes 820, ..., 832. In particular, edges 840, ..., 842 can exist only between neighboring layers of nodes. In the displayed example, there is an input layer 810 comprising only nodes 820, ..., 822 without an incoming edge, an output layer 813 comprising only nodes 831, 832 without outgoing edges, and hidden layers 811, 812 in-between the input layer 810 and the output layer 813. In general, the number of hidden layers 811, 812 can be chosen arbitrarily. In an MLP, this number is at least one. The number of nodes 820, ..., 822 within the input layer 810 usually relates to the number of input values of the artificial neural network 800, and the number of nodes 831, 832 within the output layer 813 usually relates to the number of output values of the artificial neural network 800.

In particular, a real number can be assigned as a value to every node 820, ..., 832 of the artificial neural network 800. Here, x⁽ⁿ⁾ᵢ denotes the value of the i-th node 820, ..., 832 of the n-th layer 810, ..., 813. The values of the nodes 820, ..., 822 of the input layer 810 are equivalent to the input values of the artificial neural network 800. The values of the nodes 831, 832 of the output layer 813 are equivalent to the output value of the artificial neural network 800. Furthermore, each edge 840, ..., 842 can comprise a weight being a real number. In particular, the weight is a real number within the interval [-1, 1] or within the interval [0, 1]. Here, w^{(m,n)}_{i,j} denotes the weight of the edge between the i-th node 820, ..., 832 of the m-th layer 810, ..., 813 and the j-th node 820, ..., 832 of the n-th layer 810, ..., 813. Furthermore, the abbreviation w⁽ⁿ⁾_{i,j} is defined for the weight w^{(n,n+1)}_{i,j}. In particular, to calculate the output values of the neural network 800, the input values are propagated through the neural network 800. In particular, the values of the nodes 820, ..., 832 of the (n+1)-th layer 810, ..., 813 can be calculated based on the values of the nodes 820, ..., 832 of the n-th layer 810, ..., 813 by ${x}_{j}^{\left(n+1\right)} = f \left({\sum }_{i} {x}_{i}^{\left(n\right)} {w}_{i , j}^{\left(n\right)}\right) .$

Herein, the function f is denoted as transfer function or activation function. Known transfer functions are step functions, the sigmoid functions, for example the logistic function, the generalized logistic function, the hyperbolic tangent, the arctangent function, the error function, the smoothstep function, or rectifier functions. The transfer function is for example used for normalization purposes. In particular, the values are propagated layer-wise through the neural network 800, wherein values of the input layer 810 are given by the input of the neural network 800, wherein values of the first hidden layer 811 can be calculated based on the values of the input layer 810 of the neural network 800, wherein values of the second hidden layer 812 can be calculated based on the values of the first hidden layer 811, and so forth.

In order to set the values w^{(m,n)}_{i,j} for the edges, the neural network 800 has to be trained using training data. In particular, training data comprises training input data and training output data (denoted as tᵢ). For a training step, the neural network 800 is applied to the training input data to generate calculated output data. In particular, the training data and the calculated output data comprise a number of values, said number being equal with the number of nodes of the output layer. In particular, a comparison between the calculated output data and the training data is used to recursively adapt the weights within the neural network 800 (backpropagation algorithm). In particular, the weights are changed according to ${w}_{i , j}^{′ \left(n\right)} = {w}_{i , j}^{\left(n\right)} - γ {δ}_{j}^{\left(n\right)} {x}_{i}^{\left(n\right)} ,$ wherein γ is a predefined learning rate, and the numbers δ⁽ⁿ⁾ⱼ can be recursively calculated as ${δ}_{j}^{\left(n\right)} = \left({\sum }_{k} {δ}_{k}^{\left(n+1\right)} {w}_{j , k}^{\left(n+1\right)}\right) f ′ \left({x}_{i}^{\left(n\right)}{w}_{i , j}^{\left(n\right)}\right)$ based on δ⁽ⁿ⁺¹⁾ⱼ, if the (n+1)-th layer is not the output layer 813, and ${δ}_{j}^{\left(n\right)} = \left({x}_{j}^{\left(n+1\right)}-{t}_{j}^{\left(n+1\right)}\right) f ′ \left({x}_{i}^{\left(n\right)}{w}_{i , j}^{\left(n\right)}\right) ,$ if the (n+1)-th layer is the output layer 813, wherein f' is the first derivative of the activation function, and t⁽ⁿ⁺¹⁾ⱼ is the comparison training value for the j-th node of the output layer 813.

The MLM 19 may for example be a CNN. A convolutional neural network, CNN, is an ANN that uses a convolution operation instead of general matrix multiplication in at least one of its layers.

These layers are denoted as convolutional layers. In particular, a convolutional layer performs a dot product of one or more convolution kernels with the convolutional layer's input data, wherein the entries of the one or more convolution kernel are parameters or weights that may be adapted by training. In particular, one can use the Frobenius inner product and the ReLU activation function. A convolutional neural network can comprise additional layers, for example pooling layers, fully connected layers, and/or normalization layers.

By using convolutional neural networks, the input can be processed in a very efficient way because a convolution operation based on different kernels can extract various image features so that by adapting the weights of the convolution kernel the relevant image features can be found during training. Furthermore, based on the weight-sharing in the convolutional kernels fewer parameters need to be trained, which prevents overfitting in the training phase and allows to have faster training or more layers in the network, improving the performance of the network.

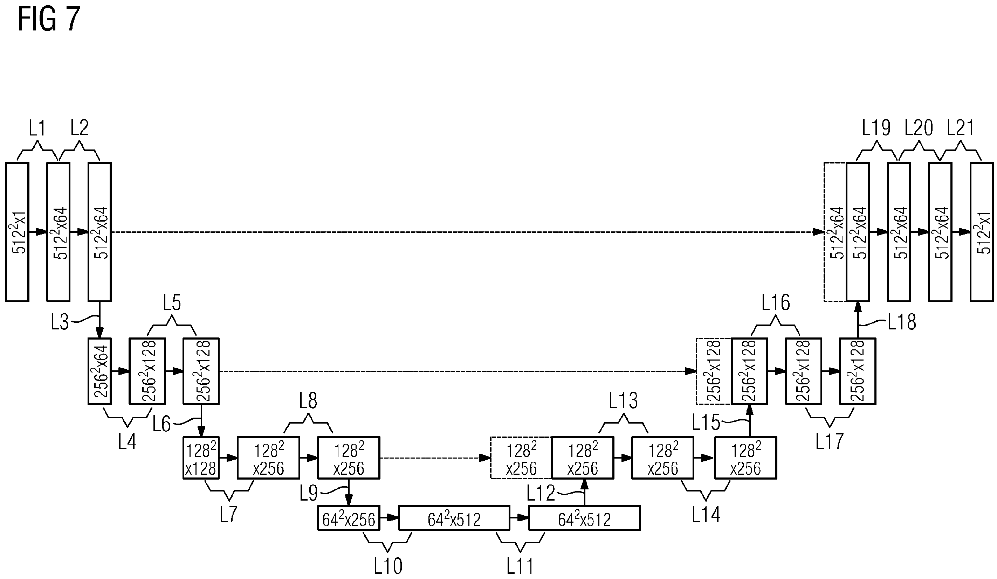

FIG 7 displays an exemplary embodiment of a convolutional neural network 700. In the displayed embodiment, the convolutional neural network 700 comprises an input node layer 710, a convolutional layer 711, a pooling layer 713, a fully connected layer 714 and an output node layer 716, as well as hidden node layers 712, 714. Alternatively, the convolutional neural network 200 can comprise several convolutional layers 711, several pooling layers 713 and/or several fully connected layers 715, as well as other types of layers. The order of the layers can be chosen arbitrarily, usually fully connected layers 715 are used as the last layers before the output layer 716.

In particular, within a convolutional neural network 700 nodes 720, 722, 724 of a node layer 710, 712, 714 can be considered to be arranged as a d-dimensional matrix or as a d-dimensional image. In particular, in the two-dimensional case the value of the node 720, 722, 724 indexed with i and j in the n-th node layer 710, 712, 714 can be denoted as x(n)[i, j]. However, the arrangement of the nodes 720, 722, 724 of one node layer 710, 712, 714 does not have an effect on the calculations executed within the convolutional neural network 700 as such, since these are given solely by the structure and the weights of the edges.

A convolutional layer 711 is a connection layer between an anterior node layer 710 with node values x(n-1) and a posterior node layer 712 with node values x(n). In particular, a convolutional layer 711 is characterized by the structure and the weights of the incoming edges forming a convolution operation based on a certain number of kernels. In particular, the structure and the weights of the edges of the convolutional layer 711 are chosen such that the values x(n) of the nodes 722 of the posterior node layer 712 are calculated as a convolution x(n) = K * x(n-1) based on the values x(n-1) of the nodes 720 anterior node layer 710, where the convolution * is defined in the two-dimensional case as ${x}^{\left(n\right)} \left[i, j\right] = \left(K∗{x}^{\left(n-1\right)}\right) \left[i, j\right] = {\sum }_{i ′} {\sum }_{j ′} K \left[i′,j′\right] ⋅ {x}^{\left(n-1\right)} \left[i-i′,j-j′\right] .$

Herein, the kernel K is a d-dimensional matrix, in the present example a two-dimensional matrix, which is usually small compared to the number of nodes 720, 722, for example a 3x3 matrix, or a 5x5 matrix. In particular, this implies that the weights of the edges in the convolution layer 711 are not independent, but chosen such that they produce said convolution equation. In particular, for a kernel being a 3x3 matrix, there are only 9 independent weights, each entry of the kernel matrix corresponding to one independent weight, irrespectively of the number of nodes 720, 722 in the anterior node layer 710 and the posterior node layer 712.

In general, convolutional neural networks 700 use node layers 710, 712, 714 with a plurality of channels, in particular, due to the use of a plurality of kernels in convolutional layers 711. In those cases, the node layers can be considered as (d+1)-dimensional matrices, the first dimension indexing the channels. The action of a convolutional layer 711 is then in a two-dimensional example defined as ${x}_{b}^{\left(n\right)} \left[i, j\right] = {\sum }_{a} \left({K}_{a , b}∗{x}_{a}^{\left(n-1\right)}\left[i, j\right]\right. = {\sum }_{a} {\sum }_{i ′} {\sum }_{j ′} {K}_{a , b} \left[i′,j′\right] ⋅ {x}_{a}^{\left(n-1\right)} \left[i-i′,j-j′\right] ,$ wherein ${x}_{a}^{\left(n\right)}$ corresponds to the a-th channel of the anterior node layer 710, ${x}_{b}^{\left(n\right)}$ corresponds to the b-th channel of the posterior node layer 712 and *K_{a,b}* corresponds to one of the kernels. If a convolutional layer 711 acts on an anterior node layer 710 with A channels and outputs a posterior node layer 712 with B channels, there are A·B independent d-dimensional kernels *K_{a,b}*.

In general, in convolutional neural networks 700 activation functions may be used. In this embodiment, ReLU (rectified linear unit) is used, with R(z) = max(0, z), so that the action of the convolutional layer 711 in the two-dimensional example is ${x}_{b}^{\left(n\right)} \left[i, j\right] = R \left({\sum }_{a} \left({K}_{a , b}∗{x}_{a}^{\left(n-1\right)}\left[i, j\right]\right.\right) = R \left({\sum }_{a} {\sum }_{i ′} {\sum }_{j ′} {K}_{a , b} \left[i′,j′\right] ⋅ {x}_{a}^{\left(n-1\right)} \left[i-i′,j-j′\right]\right) .$

It is also possible to use other activation functions, for example ELU (exponential linear unit), LeakyReLU, Sigmoid, Tanh or Softmax.

In the displayed embodiment, the input layer 710 comprises 36 nodes 720, arranged as a two-dimensional 6x6 matrix. The first hidden node layer 712 comprises 72 nodes 722, arranged as two two-dimensional 6x6 matrices, each of the two matrices being the result of a convolution of the values of the input layer with a 3x3 kernel within the convolutional layer 711. Equivalently, the nodes 722 of the first hidden node layer 712 can be interpreted as arranged as a three-dimensional 2x6x6 matrix, wherein the first dimension correspond to the channel dimension.

An advantage of using convolutional layers 711 is that spatially local correlation of the input data can exploited by enforcing a local connectivity pattern between nodes of adjacent layers, in particular by each node being connected to only a small region of the nodes of the preceding layer.

A pooling layer 713 is a connection layer between an anterior node layer 712 with node values x(n-1) and a posterior node layer 714 with node values x(n). In particular, a pooling layer 713 can be characterized by the structure and the weights of the edges and the activation function forming a pooling operation based on a non-linear pooling function f. For example, in the two-dimensional case the values x(n) of the nodes 724 of the posterior node layer 714 can be calculated based on the values x(n-1) of the nodes 722 of the anterior node layer 712 as ${x}_{b}^{\left(n\right)} \left[i, j\right] = f \left({x}_{b}^{\left(n-1\right)}\left[{id}_{1}, {jd}_{2}\right],…, {x}_{b}^{\left(n-1\right)}\left[\left(i+1\right){d}_{1}-1,\left(j+1\right){d}_{2}-1\right]\right) .$

In other words, by using a pooling layer 713, the number of nodes 722, 724 can be reduced by re-placing a number d1·d2 of neighboring nodes 722 in the anterior node layer 712 with a single node 722 in the posterior node layer 714 being calculated as a function of the values of said number of neighboring nodes. In particular, the pooling function f can be the max-function, the average or the L2-Norm. In particular, for a pooling layer 713 the weights of the incoming edges are fixed and are not modified by training.

The advantage of using a pooling layer 713 is that the number of nodes 722, 724 and the number of parameters is reduced. This leads to the amount of computation in the network being reduced and to a control of overfitting.

In the displayed embodiment, the pooling layer 713 is a max-pooling layer, replacing four neighboring nodes with only one node, the value being the maximum of the values of the four neighboring nodes. The max-pooling is applied to each d-dimensional matrix of the previous layer. In this embodiment, the max-pooling is applied to each of the two two-dimensional matrices, reducing the number of nodes from 72 to 18.

In general, the last layers of a convolutional neural network 700 may be fully connected layers 715. A fully connected layer 715 is a connection layer between an anterior node layer 714 and a posterior node layer 716. A fully connected layer 713 can be characterized by the fact that a majority, in particular, all edges between nodes 714 of the anterior node layer 714 and the nodes 716 of the posterior node layer are present, and wherein the weight of each of these edges can be adjusted individually.

In this embodiment, the nodes 724 of the anterior node layer 714 of the fully connected layer 715 are displayed both as two-dimensional matrices, and additionally as non-related nodes, indicated as a line of nodes, wherein the number of nodes was reduced for a better presentability. This operation is also denoted as flattening. In this embodiment, the number of nodes 726 in the posterior node layer 716 of the fully connected layer 715 smaller than the number of nodes 724 in the anterior node layer 714. Alternatively, the number of nodes 726 can be equal or larger.

Furthermore, in this embodiment the Softmax activation function is used within the fully connected layer 715. By applying the Softmax function, the sum the values of all nodes 726 of the output layer 716 is 1, and all values of all nodes 726 of the output layer 716 are real numbers between 0 and 1. In particular, if using the convolutional neural network 700 for categorizing input data, the values of the output layer 716 can be interpreted as the probability of the input data falling into one of the different categories.

In particular, convolutional neural networks 700 can be trained based on the backpropagation algorithm. For preventing overfitting, methods of regularization can be used, for example dropout of nodes 720, ..., 724, stochastic pooling, use of artificial data, weight decay based on the L1 or the L2 norm, or max norm constraints.

In the example of FIG 7, the MLM is a CNN, in particular, a convolutional neural network having a U-Net structure. In the displayed example, the input data to the CNN is a two-dimensional medical image comprising 512x512 pixels, every pixel comprising one intensity value. The CNN comprises convolutional layers indicated by solid, horizontal arrows, pooling layers indicating by solid arrows pointing down, and upsampling layers indicated by solid arrows pointing up. The number of the respective nodes is indicated within the boxes. Within the U-Net structure first the input images are downsampled, in particular by decreasing the size of the images and increasing the number of channels. Afterwards they are upsampled, in particular by increasing the size of the images and decreasing the number of channels, to generate a transformed image.

All except the last convolutional layers L1, L2, L4, L5, L.7, L8, L10, L11, L13, L14, L16, L17, L19, L20 use 3x3 kernels with a padding of 1, the ReLU activation function, and a number of filters or convolutional kernels that matches the number of channels of the respective node layers as indicated in FIG 7. The last convolutional layer uses a 1x1 kernel with no padding and the ReLU activation function.

The pooling layers L3, L6, L9 are max-pooling layers, replacing four neighboring nodes with only one node, the value being the maximum of the values of the four neighboring nodes. The upsampling layers L12, L15, L18 are transposed convolution layers with 3x3 kernels and stride 2, which effectively quadruple the number of nodes. The dashed horizontal arrows correspond to concatenation operations, where the output of a convolutional layer L2, L5, L8 of the downsampling branch of the U-Net structure is used as additional inputs for a convolutional layer L13, L16, L19 of the upsampling branch of the U-Net structure. This additional input data is treated as additional channels in the input node layer for the convolutional layer L13, L16, L19 of the upsampling branch.

For training the CNN, a database of 500 first medical images was used, wherein the respective segmentation mask was created based on annotations of expert radiologists. In particular, the experts determined for each of the 500 first medical images a segmentation mask for a structure of interest, where a value of 1 was assigned to pixels corresponding to the structure of interest, and a value of 0 was assigned to pixels not corresponding to the structure of interest. The database was split into training data (320 datasets), validation data (80 datasets) and test data (100 datasets). For training the CNN, the backpropagation algorithm was used based on a binary cross-entropy cost function $L \left(x, y\right) = {\sum }_{i} {\sum }_{j} BCE \left(y\left[i, j\right],M\left(x\right)\left[i, j\right]\right)$ with $BCE \left(a, b\right) : = - a log \left(b\right) \left(b\right) - \left(1-a\right) log \left(1-b\right) ,$ wherein x denotes a first medical image, y determines the corresponding segmentation mask created by the expert radiologist, and M(x) denotes the result of applying the CNN to the first input medical image x. Alternatively, one could use other cost functions like weighted binary cross entropy, Focal Loss or Dice Loss.

Based on the validation set of 80 datasets and the corresponding annotations, the best performing machine learning model out of several machine learning models (with different hyperparameters, for example number of layers, size and number of kernels, padding et cetera) was selected. The specificity and the sensitivity were determined based on the test set comprising 100 datasets and the corresponding annotations.

Independent of the grammatical term usage, individuals with male, female or other gender identities are included within the term.

## Claims

1. Computer-implemented method for denoising in X-ray imaging, wherein
- at least two noise level parameters (11) of an X-ray imaging system (1) for at least two frequency bands are received, wherein each noise level parameter (11) is associated with exactly one of the at least two frequency bands;
- a first X-ray image (10) generated by the X-ray imaging system (1) is received;
- a first denoised X-ray image (13) is generated by applying a denoising algorithm (12) depending on the at least two noise level parameters (11) to the first X-ray image (10),
- wherein applying the denoising algorithm (12) to the first X-ray image (10) comprises applying a trained machine learning model, MLM, to input data comprising the first X-ray image (10) and metadata, wherein the metadata comprises the at least two noise level parameters (11).

2. Computer-implemented method according to claim 1, wherein
- the first X-ray image (10) and the metadata are fused to generate fused input data and the MLM is applied to the fused input data; or
- image features are generated by applying a first part of the MLM to the first X-ray image (10), the image features and the metadata are fused to generate fused features, and the first denoised X-ray image (13) is generated by applying a second part of the MLM to the fused features.

3. Computer-implemented method according to one of the preceding claims, wherein
- the first X-ray image (10) corresponds to a first frame of a plurality of consecutive frames and a second X-ray image generated by the X-ray imaging system (1) is received, wherein the second X-ray image corresponds to a second frame of the plurality of consecutive frames, which succeeds the first frame;
- a difference image corresponding to a difference between the first X-ray image (10) and the second X-ray image is computed;
- a frequency decomposition is carried out to generate at least two respective variance maps of the difference image according to the at least two frequency bands;
- the denoising algorithm (12) is applied to the first X-ray image (10) depending on the at least two variance maps and/or a second denoised X-ray image is generated by applying the denoising algorithm (12) to the second X-ray image depending on the at least two variance maps.

4. Computer-implemented method according to claim 3, wherein
- each of a plurality of image regions of the second X-ray image is classified as a static region or as a dynamic region depending the at least two variance maps using the respective noise level parameter (11) as a classification threshold; and
- the denoising algorithm (12) comprises a temporal denoising step (16) with an adjustable (5) temporal denoising strength and the temporal denoising strength is adjusted depending on a result of the classification for applying the temporal denoising step (16) to the second X-ray image or to an image depending on the second X-ray image.

5. Computer-implemented method according to claim 4, wherein
- if a number of image regions classified as static regions is larger than a predefined upper threshold value, the temporal denoising strength is adjusted to a predefined upper temporal denoising strength and the temporal denoising step (16) is applied to all of the plurality of image regions according to the upper temporal denoising strength; and/or
- if the number of image regions classified as static regions is less than a predefined lower threshold value, which is less than the upper threshold value, the temporal denoising strength is adjusted to a predefined lower temporal denoising strength, which is smaller than the predefined upper temporal denoising strength, and the temporal denoising step (16) is applied to all of the plurality of image regions according to the lower temporal denoising strength.

6. Computer-implemented method according to one of claims 4 or 5, wherein if the number of image regions classified as static regions is less than the upper threshold value and larger than the lower threshold value, then
- the temporal denoising step (16) is applied to all static image regions according to the upper temporal denoising strength; and
- the temporal denoising step (16) is applied to all dynamic image regions according to the lower temporal denoising strength or the temporal denoising step (16) is not applied to any of the dynamic image regions.

7. Computer-implemented method according to claim 6, wherein the denoising algorithm (12) comprises a spatial denoising step (15) with an adjustable (5) spatial denoising strength and
- the spatial denoising step (15) is applied to all dynamic image regions according to a predefined upper spatial denoising strength;
- the spatial denoising step (15) is applied to all static image regions according to a predefined lower spatial denoising strength, which is less than the upper denoising strength; and
- the upper spatial denoising strength and the lower spatial denoising strength are adjusted such that an overall denoising strength of the temporal denoising step (16) and the spatial denoising step (15) is constant for all image regions of the plurality of image regions.

8. Computer-implemented method according to one of the preceding claims, wherein
- a set of imaging parameters (18, 19, 20) of the X-ray imaging system (1) corresponding to the generation of first X-ray image (10) is received;
- the at least two noise level parameters (11) are determined depending on the set of imaging parameters (18, 19, 20).

9. Computer-implemented method according to claim 8, wherein
- a plurality of variance stabilized reference X-ray images generated by the X-ray imaging system (1) according to respective different sets of imaging parameters (18, 19, 20) is received;
- an average reference image is generated by averaging the reference X-ray images;
- for each of the reference X-ray images, a respective difference reference image corresponding to a difference between the respective reference X-ray image and the average reference image is computed;
- for each of the difference reference images, a frequency decomposition is carried out according to the at least two frequency bands;
- for each of the at least two frequency bands, the respective noise level parameter (11) is computed depending on the noise variance in the respective frequency band of the difference reference images.

10. Computer-implemented method according to one of claims 8 or 9, wherein the set of imaging parameters (18, 19, 20) comprises
- a peak kilovoltage of an X-ray source (3) of the X-ray imaging system (1); and/or
- a tube current of the X-ray source (3) of the X-ray imaging system (1); and/or
- an X-ray pulse duration; and/or
- a filter material and/or filter thickness of an X-ray filter of the X-ray imaging system (1); and/or
- a collimator opening size of an X-ray collimator of the X-ray imaging system (1); and/or
- a position and/or orientation of the X-ray source (3); and/or
- a position and/or orientation of the X-ray detector (4); and/or
- a position and/or angulation of a C-arm (2) carrying the X-ray source (3) and the X-ray detector (4); and/or
- a position and/or orientation of a patient table (5) or a part of the patient table (5); and/or
- an effective thickness of an imaged object (6); and/or
- a gain factor of the X-ray detector (4); and/or
- a status parameter of an anti-scattering grid of the X-ray imaging system (1); and/or
- a sensitivity of detector pixels of the X-ray detector (4).

11. Computer-implemented training method for supervised training of a denoising algorithm (12) for denoising in X-ray imaging, the denoising algorithm being a machine learning model, MLM, wherein
- at least two noise level parameters (11) of an X-ray imaging system (1) for at least two frequency bands are received, wherein each noise level parameter (11) is associated with exactly one of the at least two frequency bands; and
- a loss function for the supervised training depends on the at least two noise level parameters (11) and
- the at least two noise level parameters (11) are passed to the MLM as auxiliary metadata during training.

12. Computer-implemented training method according to claim 11, wherein the loss function comprises a weighted sum of errors according to the at least two frequency bands, wherein respective weighting factors of the weighted sum depend on the at least two noise level parameters (11).

13. Data processing system (7, 9) adapted to carry out a computer-implemented method according to one of claims 1 to 10 and/or a computer-implemented training method according to one of claims 11 or 12.

14. X-ray imaging system (1) comprising
- an X-ray source (3), an X-ray detector (4), and a control system (7), which is configured to control the X-ray source (3) and the X-ray detector (4) to generate a first X-ray image (10);
- a data processing system (7, 9) (7, 9), which is adapted to carry out a computer-implemented method according to one of claims 1 to 10; and
- a display device (8), wherein the control system (7) is configured to control the display device to display the first denoised X-ray image (13).

15. Computer program product comprising
- instructions, which, when executed by a data processing system (7, 9), cause the data processing system (7, 9) to carry out a computer-implemented method according to one of claims 1 to 10; and/or
- further instructions, which, when executed by a further data processing system (7, 9), cause the further data processing system (7, 9) to carry out a computer-implemented training method according to one of claims 11 or 12.

## Patentansprüche

1. Computerimplementiertes Verfahren zum Mindern des Rauschens in der Röntgenbildgebung, wobei
- mindestens zwei Rauschpegelparameter (11) eines Röntgenbildgebungssystems (1) für mindestens zwei Frequenzbänder empfangen werden, wobei jeder Rauschpegelparameter (11) genau einem der mindestens zwei Frequenzbänder zugeordnet ist;
- ein erstes Röntgenbild (10), das durch das Röntgenbildgebungssystem (1) erzeugt wird, empfangen wird;
- durch Anwenden eines Rauschminderungsalgorithmus (12) in Abhängigkeit von den mindestens zwei Rauschpegelparametern (11) auf das erste Röntgenbild (10) ein erstes rauschgemindertes Röntgenbild (13) erzeugt wird,
- wobei das Anwenden des Rauschminderungsalgorithmus (12) auf das erste Röntgenbild (10) das Anwenden eines trainierten Modells des maschinellen Lernens, MLM, auf Eingangsdaten, die das erste Röntgenbild (10) und Metadaten umfassen, wobei die Metadaten die mindestens zwei Rauschpegelparameter (11) umfassen, umfasst.

2. Computerimplementiertes Verfahren nach Anspruch 1, wobei
- das erste Röntgenbild (10) und die Metadaten fusioniert werden, um fusionierte Eingangsdaten zu erzeugen, und das MLM auf die fusionierten Eingangsdaten angewendet wird; oder
- durch Anwenden eines ersten Teils des MLM auf das erste Röntgenbild (10) Bildmerkmale erzeugt werden, wobei die Bildmerkmale und die Metadaten fusioniert werden, um fusionierte Merkmale zu erzeugen, und das erste rauschgeminderte Röntgenbild (13) durch Anwenden eines zweiten Teils des MLM auf die fusionierten Merkmale erzeugt wird.

3. Computerimplementiertes Verfahren nach einem der vorhergehenden Ansprüche, wobei
- das erste Röntgenbild (10) einem ersten Einzelbild einer Vielzahl von aufeinanderfolgenden Einzelbildern entspricht und ein durch das Röntgenbildgebungssystem (1) erzeugtes zweites Röntgenbild empfangen wird, wobei das zweite Röntgenbild einem zweiten Einzelbild der Vielzahl von aufeinanderfolgenden Einzelbildern, das dem ersten Einzelbild folgt, entspricht;
- ein Differenzbild, das einer Differenz zwischen dem ersten Röntgenbild (10) und dem zweiten Röntgenbild entspricht, berechnet wird;
- eine Frequenzzerlegung durchgeführt wird, um in Übereinstimmung mit den mindestens zwei Frequenzbändern mindestens zwei jeweilige Varianzkarten des Differenzbilds zu erzeugen;
- der Rauschminderungsalgorithmus (12) in Abhängigkeit von den mindestens zwei Varianzkarten auf das erste Röntgenbild (10) angewendet wird und/oder durch Anwenden des Rauschminderungsalgorithmus (12) auf das zweite Röntgenbild in Abhängigkeit von den mindestens zwei Varianzkarten ein zweites rauschgemindertes Röntgenbild erzeugt wird.

4. Computerimplementiertes Verfahren nach Anspruch 3, wobei
- jeder einer Vielzahl von Bildbereichen des zweiten Röntgenbilds in Abhängigkeit von den mindestens zwei Varianzkarten unter Verwendung des jeweiligen Rauschpegelparameters (11) als ein Klassifizierungsschwellenwert als ein statisches Gebiet oder als ein dynamisches Gebiet klassifiziert wird; und
- der Rauschminderungsalgorithmus (12) einen Schritt (16) der zeitlichen Rauschminderung mit einer einstellbaren (5) Stärke der zeitlichen Rauschminderung umfasst und die Stärke der zeitlichen Rauschminderung in Abhängigkeit von einem Ergebnis der Klassifizierung zum Anwenden des Schritts (16) der zeitlichen Rauschminderung auf das zweite Röntgenbild oder auf ein Bild in Abhängigkeit von dem zweiten Röntgenbild eingestellt wird.

5. Computerimplementiertes Verfahren nach Anspruch 4, wobei
- die Stärke der zeitlichen Rauschminderung auf eine vordefinierte höhere Stärke der zeitlichen Rauschminderung eingestellt wird und der Schritt (16) der zeitlichen Rauschminderung auf alle der mehreren Bildbereiche in Übereinstimmung mit der höheren Stärke der zeitlichen Rauschminderung angewendet wird, falls eine Anzahl von als statische Bereiche klassifizierten Bildbereichen größer als ein vordefinierter oberer Schwellenwert ist; und/oder
- die Stärke der zeitlichen Rauschminderung auf eine vordefinierte niedrigere Stärke der zeitlichen Rauschminderung, die kleiner als die vordefinierte höhere Stärke der zeitlichen Rauschminderung ist, eingestellt wird und der Schritt (16) der zeitlichen Rauschminderung auf alle der Vielzahl von Bildbereichen in Übereinstimmung mit der niedrigeren Stärke der zeitlichen Rauschminderung angewendet wird, falls die Anzahl der als statische Bereiche klassifizierten Bildbereiche kleiner als ein vordefinierter unterer Schwellenwert ist, der kleiner als der obere Schwellenwert ist.

6. Computerimplementiertes Verfahren nach einem der Ansprüche 4 oder 5, wobei, falls die Anzahl von als statische Gebiete klassifizierten Bildbereichen kleiner als der obere Schwellenwert und größer als der untere Schwellenwert ist:
- der Schritt (16) der zeitlichen Rauschminderung auf alle statischen Bildbereiche in Übereinstimmung mit der höheren Stärke der zeitlichen Rauschminderung angewendet wird; und
- der Schritt (16) der zeitlichen Rauschminderung auf alle dynamischen Bildbereiche in Übereinstimmung mit der niedrigeren Stärke der zeitlichen Rauschminderung angewendet wird oder der Schritt (16) der zeitlichen Rauschminderung auf keinen der dynamischen Bildbereiche angewendet wird.

7. Computerimplementiertes Verfahren nach Anspruch 6, wobei der Rauschminderungsalgorithmus (12) einen Schritt (15) der räumlichen Rauschminderung mit einer einstellbaren (5) Stärke der räumlichen Rauschminderung umfasst und
- der Schritt (15) der räumlichen Rauschminderung auf alle dynamischen Bildbereiche in Übereinstimmung mit einer vordefinierten höheren Stärke der räumlichen Rauschminderung angewendet wird;
- der Schritt (15) der räumlichen Rauschminderung auf alle statischen Bildbereiche in Übereinstimmung mit einer vordefinierten niedrigeren Stärke der räumlichen Rauschminderung, die kleiner als die Stärke der höheren Rauschminderung ist, angewendet wird; und
- die höhere Stärke der räumlichen Rauschminderung und die niedrigere Stärke der räumlichen Rauschminderung so eingestellt werden, dass eine Gesamtrauschminderungsstärke des Schritts (16) der zeitlichen Rauschminderung und des Schritts (15) der räumlichen Rauschminderung für alle Bildbereiche der mehreren Bildbereiche konstant ist.

8. Computerimplementiertes Verfahren nach einem der vorhergehenden Ansprüche, wobei
- ein Satz von Bildgebungsparametern (18, 19, 20) des Röntgenbildgebungssystems (1), der der Erzeugung des ersten Röntgenbilds (10) entspricht, empfangen wird;
- die mindestens zwei Rauschpegelparameter (11) in Abhängigkeit von dem Satz von Bildgebungsparametern (18, 19, 20) bestimmt werden.

9. Computerimplementiertes Verfahren nach Anspruch 8, wobei
- mehrere varianzstabilisierte Referenzröntgenbilder, die durch das Röntgenbildgebungssystem (1) in Übereinstimmung mit jeweiligen unterschiedlichen Sätzen von Bildgebungsparametern (18, 19, 20) erzeugt werden, empfangen werden;
- durch Mitteln der Referenzröntgenbilder ein durchschnittliches Referenzbild erzeugt wird;
- für jedes der Referenzröntgenbilder ein jeweiliges Differenzreferenzbild, das einer Differenz zwischen dem jeweiligen Referenzröntgenbild und dem durchschnittlichen Referenzbild entspricht, berechnet wird;
- für jedes der Differenzreferenzbilder in Übereinstimmung mit den mindestens zwei Frequenzbändern eine Frequenzzerlegung durchgeführt wird;
- für jedes der mindestens zwei Frequenzbänder in Abhängigkeit von der Rauschvarianz im jeweiligen Frequenzband der Differenzreferenzbilder der jeweilige Rauschpegelparameter (11) berechnet wird.

10. Computerimplementiertes Verfahren nach einem der Ansprüche 8 oder 9, wobei der Satz von Bildgebungsparametern (18, 19, 20) Folgendes umfasst:
- eine Spitzen-Kilovoltspannung einer Röntgenquelle (3) des Röntgenbildgebungssystems (1); und/oder
- einen Röhrenstrom der Röntgenquelle (3) des Röntgenbildgebungssystems (1); und/oder
- eine Röntgenpulsdauer; und/oder
- ein Filtermaterial und/oder eine Filterdicke eines Röntgenfilters des Röntgenbildgebungssystems (1); und/oder
- eine Kollimatoröffnungsgröße eines Röntgenkollimators des Röntgenbildgebungssystems (1); und/oder
- eine Position und/oder Orientierung der Röntgenquelle (3); und/oder
- eine Position und/oder Orientierung des Röntgendetektors (4); und/oder
- eine Position und/oder Winkelstellung eines C-Bogens (2), der die Röntgenquelle (3) und den Röntgendetektor (4) trägt; und/oder
- eine Position und/oder Orientierung eines Patiententischs (5) oder eines Teils des Patiententischs (5); und/oder
- eine effektive Dicke eines abgebildeten Objekts (6); und/oder
- einen Verstärkungsfaktor des Röntgendetektors (4); und/oder
- einen Statusparameter eines Antistreugitters des Röntgenbildgebungssystems (1); und/oder
- eine Empfindlichkeit der Detektorpixel des Röntgendetektors (4).

11. Computerimplementiertes Trainingsverfahren zum überwachten Training eines Rauschminderungsalgorithmus (12) zum Mindern des Rauschens in der Röntgenbildgebung, wobei der Rauschminderungsalgorithmus ein Modell des maschinellen Lernens, MLM, ist, wobei
- mindestens zwei Rauschpegelparameter (11) eines Röntgenbildgebungssystems (1) für mindestens zwei Frequenzbänder empfangen werden, wobei jeder Rauschpegelparameter (11) genau einem der mindestens zwei Frequenzbänder zugeordnet ist; und
- eine Verlustfunktion für das überwachte Training von den mindestens zwei Rauschpegelparametern (11) abhängt und
- die mindestens zwei Rauschpegelparameter (11) während des Trainings als Hilfsmetadaten an das MLM übergeben werden.

12. Computerimplementiertes Trainingsverfahren nach Anspruch 11, wobei die Verlustfunktion eine gewichtete Summe von Fehlern in Übereinstimmung mit den mindestens zwei Frequenzbändern umfasst, wobei jeweilige Gewichtungsfaktoren der gewichteten Summe von den mindestens zwei Rauschpegelparametern (11) abhängen.

13. Datenverarbeitungssystem (7, 9), das dazu ausgelegt ist, ein computerimplementiertes Verfahren nach einem der Ansprüche 1 bis 10 und/oder ein computerimplementiertes Trainingsverfahren nach einem der Ansprüche 11 oder 12 durchzuführen.

14. Röntgenbildgebungssystem (1), das Folgendes umfasst:
- eine Röntgenquelle (3), einen Röntgendetektor (4) und ein Steuersystem (7), das dazu ausgelegt ist, die Röntgenquelle (3) und den Röntgendetektor (4) zum Erzeugen eines ersten Röntgenbilds (10) zu steuern;
- ein Datenverarbeitungssystem (7, 9) (7, 9), das zum Durchführen eines computerimplementierten Verfahrens nach einem der Ansprüche 1 bis 10 ausgelegt ist; und
- eine Anzeigevorrichtung (8), wobei das Steuersystem (7) dazu ausgelegt ist, die Anzeigevorrichtung zum Anzeigen des ersten rauschgeminderten Röntgenbilds (13) zu steuern.

15. Computerprogrammprodukt, das Folgendes umfasst:
- Anweisungen, die, wenn sie durch ein Datenverarbeitungssystem (7, 9) ausgeführt werden, veranlassen, dass das Datenverarbeitungssystem (7, 9) ein computerimplementiertes Verfahren nach einem der Ansprüche 1 bis 10 ausführt; und/oder
- weitere Anweisungen, die, wenn sie durch ein weiteres Datenverarbeitungssystem (7, 9) ausgeführt werden, veranlassen, dass das weitere Datenverarbeitungssystem (7, 9) ein computerimplementiertes Trainingsverfahren nach einem der Ansprüche 11 oder 12 ausführt.

## Revendications

1. Procédé mis en œuvre par ordinateur de débruitage en imagerie par rayons X, dans lequel
- au moins deux paramètres (11) de niveau de bruit d'un système (1) d'imagerie par rayons X sont reçus pour au moins deux bandes de fréquence, dans lequel chaque paramètre (11) de niveau de bruit est associé à exactement l'une des au moins deux bandes de fréquence ;
- une première image (10) de rayons X créée par le système (1) d'imagerie par rayons X est reçue ;
- une première image (13) débruitée de rayons X est créée en appliquant un algorithme (12) de débruitage en fonction des au moins deux paramètres (11) de niveau de bruit à la première image (10) de rayons X,
- dans lequel appliquer l'algorithme (12) de débruitage à la première image (10) de rayons X comprend appliquer un modèle entraîné d'apprentissage automatique, MLM, à des données d'entrée comprenant la première image (10) de rayons X et une métadonnée, dans lequel la métadonnée comprend les au moins deux paramètres (11) de niveau de bruit.

2. Procédé mis en œuvre par ordinateur suivant la revendication 1, dans lequel
- la première image (10) de rayons X et la métadonnée sont fusionnées pour créer une donnée d'entrée fusionnée et le MLM est appliqué à la donnée d'entrée fusionnée ; ou
- des caractéristiques d'image sont créées en appliquant une première partie du MLM à la première image (10) de rayons X, les caractéristiques d'image et la métadonnée sont fusionnées pour créer des caractéristiques fusionnées, et la première image (13) débruitée de rayons X est créée en appliquant une deuxième partie du MLM aux caractéristiques fusionnées.

3. Procédé mis en œuvre par ordinateur suivant l'une des revendications précédentes, dans lequel
- la première image (10) de rayons X correspond à une première trame d'une pluralité de trames consécutives et une deuxième image de rayons X créée par le système (1) d'imagerie par rayons X est reçue, dans lequel la deuxième image de rayons X correspond à une deuxième trame de la pluralité de trames consécutives, qui succède à la première trame ;
- une image de différence correspondant à une différence entre la première image (10) de rayons X et la deuxième image de rayons X est calculée ;
- une décomposition en fréquence est effectuée pour créer au moins deux cartes de variance respectives de l'image de différence suivant les au moins deux bandes de fréquence ;
- l'algorithme (12) de débruitage est appliqué à la première image (10) de rayons X en fonction des au moins deux cartes de variance et/ou une deuxième image débruitée de rayons X est créée en appliquant l'algorithme (12) de débruitage à la deuxième image de rayons X en fonction des au moins deux cartes de variance.

4. Procédé mis en œuvre par ordinateur suivant la revendication 3, dans lequel
- chacune de la pluralité de régions d'image de la deuxième image de rayons X est classée comme une région statique ou comme une région dynamique en fonction des au moins deux cartes de variance en utilisant le paramètre (11) respectif de niveau de bruit comme seuil de classement ; et
- l'algorithme (12) de débruitage comprend un stade (16) temporel de débruitage ayant une force réglable (5) de débruitage temporel et la force de débruitage temporel est réglée en fonction d'un résultat du classement pour appliquer le stade (16) de débruitage temporel à la deuxième image de rayons X ou à une image dépendante de la deuxième image de rayons X.

5. Procédé mis en œuvre par ordinateur suivant la revendication 4, dans lequel,
- si un nombre de régions d'image classées comme régions statiques est plus grand qu'une valeur de seuil supérieure définie à l'avance, la force de débruitage temporel est réglée à une force de débruitage temporel supérieure définie à l'avance et le stade (16) de débruitage temporel est appliqué à toute la pluralité de régions d'image suivant la force de débruitage temporel supérieure ; et/ou
- si le nombre de régions d'image classées comme régions statiques est plus petit qu'une valeur de seuil inférieure définie à l'avance, qui est inférieure à la valeur de seuil supérieure, la force de débruitage temporel est réglée à une force de débruitage temporel inférieure définie à l'avance, qui est plus petite que la force de débruitage temporel supérieure définie à l'avance, et le stade (16) de débruitage temporel est appliqué à toute la pluralité de régions d'image suivant la force de débruitage temporel inférieure.

6. Procédé mis en œuvre par ordinateur suivant l'une des revendications 4 ou 5, dans lequel, si le nombre de régions d'image classées comme régions statiques est plus petit que la valeur de seuil supérieure et plus grande que la valeur de seuil inférieure,
- le stade (16) de débruitage temporel est appliqué à toutes les régions d'image statique suivant la force de débruitage temporel supérieure ; et
- le stade (16) de débruitage temporel est appliqué à toutes les régions d'image dynamiques suivant la force de débruitage temporel inférieure ou le stade (16) de débruitage temporel n'est pas appliqué à l'une quelconque des régions d'image dynamiques.

7. Procédé mis en œuvre par ordinateur suivant la revendication 6, dans lequel l'algorithme (12) de débruitage comprend un stade (15) de débruitage spatial ayant une force de débruitage spatial réglable (5) et
- le stade (15) de débruitage spatial est appliqué à toutes les régions d'image dynamiques suivant une force de débruitage spatial supérieure définie à l'avance ;
- le stade (15) de débruitage spatial est appliqué à toutes les régions d'image statiques suivant une force de débruitage spatial inférieure définie à l'avance, qui est plus petite que la force de débruitage supérieure ; et
- la force de débruitage spatial supérieure et la force de débruitage spatial inférieure sont réglées de manière à ce qu'une force de débruitage d'ensemble du stade (16) de débruitage temporel et du stade (15) de débruitage spatial soit constante pour toutes les régions d'image de la pluralité de régions d'image.

8. Procédé mis en œuvre par ordinateur suivant l'une des revendications précédentes, dans lequel
- un ensemble de paramètres (18, 19, 20) d'imagerie du système (1) d'imagerie par rayons X correspondant à la création d'une première image (10) de rayons X est reçu ;
- les au moins deux paramètres (11) de niveau de bruit sont déterminés en fonction de l'ensemble de paramètres (18, 19, 20) d'imagerie.

9. Procédé mis en œuvre par ordinateur suivant la revendication 8, dans lequel
- une pluralité d'images de rayons X de référence stabilisées en variance créées par le système (1) d'imagerie par rayons X est reçue suivant des jeux différents de paramètres (18, 19, 20) d'imagerie ;
- une image de référence moyenne est créée en faisant la moyenne des images de rayons X de référence ;
- pour chacune des images de rayons X de référence, une image de référence de différence respective correspondant à une différence entre l'image de rayons X de référence respective et l'image de référence moyenne est calculée ;
- pour chacune des images de référence de différence, on effectue une décomposition en fréquence suivant les au moins deux bandes de fréquence ;
- pour chacune des au moins deux bandes de fréquence, le paramètre (11) de niveau de bruit respectif est calculé en fonction de la variance de bruit dans la bande de fréquence respective des images de référence de différence.

10. Procédé mis en œuvre par ordinateur suivant l'une des revendications 8 ou 9, dans lequel l'ensemble de paramètres (18, 19, 20) d'imagerie comprend
- un kilovoltage pic d'une source (3) de rayons X du système (1) d'imagerie par rayons X ; et/ou
- un courant de tube de la source (3) de rayons X du système (1) d'imagerie par rayons X ; et/ou
- une durée d'impulsion de rayons X ; et/ou
- un matériau de filtre et/ou une épaisseur de filtre d'un filtre de rayons X du système (1) d'imagerie par rayons X ; et/ou
- une dimension d'ouverture d'un collimateur de rayons X du système (1) d'imagerie par rayons X ; et/ou
- une position et/ou une orientation de la source (3) de rayons X ; et/ou
- une position et/ou une orientation du détecteur (4) de rayons X ; et/ou
- une position et/ou un angle du bras (2) en C portant la source (3) de rayons X et le détecteur (4) de rayons X ; et/ou
- une position et/ou une orientation d'une table (5) de patient ou d'une partie de la table (5) de patient ; et/ou
- une épaisseur effective d'un objet (6), dont on prend une image ; et/ou
- un facteur de gain du détecteur (4) de rayons X ; et/ou
- un paramètre de statut d'une grille d'anti-dispersion du système (1) d'imagerie par rayons X ; et/ou
- une sensibilité de pixels du détecteur (4) de rayons X.

11. Procédé d'apprentissage mis en œuvre par ordinateur pour un apprentissage supervisé d'un algorithme (12) de débruitage afin de débruiter une imagerie par rayons X, l'algorithme de débruitage étant un modèle d'apprentissage automatique, MLM, dans lequel
- au moins deux paramètres (11) de niveau de bruit d'un système (1) d'imagerie par rayons X pour au moins deux bandes de fréquence sont reçus, dans lequel chaque paramètre (11) de niveau de bruit est associé à exactement l'une des au moins deux bandes de fréquence ; et
- une fonction de perte pour l'apprentissage supervisé dépend des au moins deux paramètres (11) de niveau de bruit et
- les au moins deux paramètres (11) de niveau de bruit sont passés au MLM comme métadonnées auxiliaires pendant l'apprentissage.

12. Procédé d'apprentissage mis en œuvre par ordinateur suivant la revendication 11, dans lequel la fonction de perte comprend une somme pondérée d'erreurs suivant les au moins deux bandes de fréquence, dans lequel des facteurs respectifs de pondération de la somme pondérée dépendent des au moins deux paramètres (11) de niveau de bruit.

13. Système (7, 9) de traitement de données propre à effectuer un procédé mis en œuvre par ordinateur suivant l'une des revendications 1 à 10 et/ou un procédé d'apprentissage mis en œuvre par ordinateur suivant l'une des revendications 11 ou 12.

14. Système (1) d'imagerie par rayons X comprenant
- une source (3) de rayons X, un détecteur (4) de rayons X et un système (7) de commande, qui est configuré pour commander la source (3) de rayons X et le détecteur (4) de rayons X, afin de créer une première image (10) de rayons X ;
- un système (7, 9) de traitement de données, qui est propre à effectuer un procédé mis en œuvre par ordinateur suivant l'une des revendications 1 à 10 ; et
- un dispositif (8) d'affichage, dans lequel le système (7) de commande est configuré pour commander le dispositif d'affichage afin d'afficher la première image (13) de rayons X débruitée.

15. Produit de programme d'ordinateur comprenant
- des instructions qui, lorsqu'elles sont exécutées par un système (7, 9) de traitement de données, font que le système (7, 9) de traitement de données exécute un procédé mis en œuvre par ordinateur suivant l'une des revendications 1 à 10 ; et/ou
- d'autres instructions qui, lorsqu'elles sont exécutées par un autre système (7, 9) de traitement de données, font que l'autre système (7, 9) de traitement de données exécute un procédé d'apprentissage mis en œuvre par ordinateur suivant l'une des revendications 11 ou 12.
